# EUROPEAN PATENT APPLICATION

(11) **EP 4 378 422 A1**
(43) Date of publication of application: **05.06.2024**
(21) Application number: 22847865.7
(22) Date of filing: 28.03.2022
(51) Int. Cl.: A61F 2/24

(54) **CRIMPER FOR COMPRESSING INTERVENTIONAL INSTRUMENT, AND LOADING METHOD FOR INTERVENTIONAL INSTRUMENT**

(30) Priority: 28.07.2021 CN 202110857607
(71) Applicant: Venus MedTech (HangZhou), Inc., Hangzhou, Zhejiang 310052 (CN)
(72) Inventor: WANG, Jian, Hangzhou, Zhejiang 310052 (CN); WANG, Xiang, Hangzhou, Zhejiang 310052 (CN)
(74) Representative: Zenz Patentanwälte Partnerschaft mbB
(86) International application number: PCT/CN2022/083448
(87) International publication number: WO 2023/005245

(57) **Abstract**

A crimper (100) for crimping an interventional instrument (200) and a method for loading an interventional instrument (200). The crimper (100) includes a housing (10) with a through instrument channel (11); a plurality of force-applying blocks (20) which are movably installed in the housing (10) and distributed around the instrument channel (11), the plurality of force-applying blocks (20) have relative gathered and separated states and are configured to contract and expand the instrument channel (11) during switching the states; and a ring gear (40) which is rotatably matched with the housing (10) and configured to synchronously drive the plurality of force-applying blocks (20) to switch the states.

## Description

### TECHNICAL FIELD

The present disclosure relates to the technical field of medical devices, and in particular to a crimper for crimping an interventional instrument.

### DESCRIPTION OF THE PRIOR ART

In order to facilitate delivery within the human body, an interventional instrument needs to be radially compressed by a crimper before surgery to obtain a smaller radial size. After being compressed, it is loaded into a delivery system and delivered to the treatment site in the human body in a compressed state, and then expands to a functional size at the desired site.

The existing crimpers include a plurality of force-applying components that act on the interventional instrument. The relative movement of the force-applying components can radially compress the interventional instrument. For example, an existing crimper includes a plurality of slide blocks used as the force-applying components. However, due to structural limitations, the shapes of the slide blocks are different, which results in cumbersome alignment and low efficiency in assembly.

### SUMMARY OF THE DISCLOSURE

In order to solve the above technical problems, the present disclosure discloses a crimper for crimping an interventional instrument, including:
a housing with a through instrument channel;
a plurality of force-applying blocks which are movably installed in the housing and distributed around the instrument channel, the plurality of force-applying blocks have relative gathered and separated states and are configured to contract and expand the instrument channel during switching the states; and
a ring gear which is rotatably matched with the housing and configured to synchronously drive the plurality of force-applying blocks to switch the states.

In the following, several alternatives are provided, but merely as further additions or preferences, instead of as additional limitations to the above-mentioned technical solution. Without technical or logical contradiction, the alternatives can be combined with the above-mentioned technical solution, individually or in combination. Optionally, the ring gear is configured to synchronously drive the plurality of force-applying blocks to switch between the gathered state and the separated state based on meshing transmission.

Optionally, the crimper further includes transmission mechanisms, and each transmission mechanism is connected between the ring gear and the corresponding force-applying block in a transmission manner.

Optionally, the transmission mechanism includes:
a rack(s) provided on the corresponding force-applying block; and
a transmission gear(s) rotatably installed in the housing, wherein each rack meshes with the ring gear through one or more transmission gears.

Optionally, a rotation axis of the ring gear and an extension direction of the instrument channel are parallel to each other.

Optionally, a rotation axis of the transmission gear and the rotation axis of the ring gear are parallel to each other.

Optionally, the ring gear includes an annular portion and driving teeth distributed on the annular portion in a transmission fit with the force-applying blocks, and the driving teeth are configured as at least one of the followings:
inner teeth distributed on an inner periphery of the annular portion;
outer teeth distributed on an outer periphery of the annular portion; and
side teeth distributed on an axial end surface of the annular portion.

Optionally, the number of the driving teeth is an integer multiple of the number of the force-applying blocks.

Optionally, the number of the driving teeth is 8 times the number of the force-applying blocks.

Optionally, the ring gear includes an annular portion, and the annular portion is provided with a plurality of weight-reducing grooves in a circumferential direction of the ring gear.

Optionally, the ring gear surrounds an outer periphery of the plurality of force-applying blocks.

Optionally, the rack and the ring gear are offset from each other in an extension direction of the instrument channel.

Optionally, two racks are fixed on each force-applying block and arranged in parallel, and the two racks are respectively located on two sides of the ring gear in an extension direction of the instrument channel.

Optionally, the transmission gear uses 1 to 3-stage gear transmission, and depending on transmission sequence, an initial-stage gear meshes with the ring gear, and a terminal-stage gear meshes with the rack.

Optionally, multiple groups of transmission gears are provided with each group corresponding to a force-applying block.

Optionally, each group of transmission gear includes a first gear and a second gear coaxially fixed with the first gear, and the first gear meshes with the ring gear, and the second gear meshes with the rack on the corresponding force-applying block.

Optionally, for each force-applying block, each rack is configured with a second gear, or all racks are configured with a common second gear.

Optionally, a reference diameter of the first gear is D1, a reference diameter of the second gear is D2, and D1: D2 = 1: (0.3 to 0.7).

Optionally, an axial length of the first gear is L1, an axial length of the second gear is L2, and L1 : L2 = (3 to 6) : 1.

Optionally, a transmission ratio between the first gear and the ring gear is in a range of 1: (5 to 15); and
a transmission ratio between the second gear and the ring gear is in a range of 1 : (5 to 15).

Optionally, the transmission ratio between the first gear and the ring gear is 1: 9.6; and the transmission ratio between the second gear and the ring gear is 1: 9.6.

Optionally, the transmission ratio between the first gear and the ring gear is 3: 26; and
the transmission ratio between the second gear and the ring gear is 3: 26.

Optionally, the rack(s) and the corresponding force-applying block are formed in one or separate pieces.

Optionally, the interventional instrument is a heart valve prosthesis.

Optionally, the crimper further includes a driving mechanism for driving the ring gear to rotate.

Optionally, the driving mechanism uses at least one of the following modes:
mode A: the driving mechanism is a driving handle indirectly or directly connected to the ring gear; and
mode B: the driving mechanism is an electrically-operated component connected to the ring gear in a transmission manner.

Optionally, the electrically-operated component is installed on the housing to drive the ring gear to rotate relative to the housing.

Optionally, the electrically-operated component is a motor.

Optionally, an operation window is opened in the housing, and at least a part of the ring gear is exposed to the operation window and connected to the driving handle.

Optionally, at least a part of the ring gear is a connection portion exposed to the operation window, and the driving handle is detachably fixed to the connection portion.

Optionally, a central angle corresponding to a stroke of the driving handle is in a range of 30 degrees to 120 degrees.

Optionally, the central angle corresponding to a stroke of the driving handle is in a range of 30 degrees to 60 degrees.

Optionally, the central angle corresponding to a stroke of the driving handle is 40 degrees.

Optionally, the driving handle includes a holding portion and a snapping portion, and the ring gear is provided with a snapping slot that matches the snapping portion.

Optionally, the driving handle has a rod-shaped structure, one end of the driving handle is connected to the ring gear, and the other end extends away from the instrument channel.

Optionally, in process of the force-applying blocks switching from the gathered state to the separated state, two adjacent force-applying blocks are always against each other in a circumferential direction of the instrument channel.

Optionally, each force-applying block has a bent portion at one end toward the instrument channel;
in a circumferential direction of the instrument channel, the bent portion has a first edge surface on an outside and a second edge surface on an inside; and
a wall of the instrument channel is surrounded by intersections of the first edge surfaces and the second edge surfaces of the force-applying blocks.

Optionally, in the circumferential direction of the instrument channel, the first edge surface of one of two adjacent force-applying blocks is in contact with the second edge surface of the other force-applying block.

Optionally, in the circumferential direction of the instrument channel, the second edge surface of one of two adjacent force-applying blocks covers a part of the first edge surface of the other force-applying block, and the other part of the first edge surface exposed to the instrument channel is configured as an action surface; and
in process of the force-applying blocks switching from the gathered state to the separated state, a length of the action surface in the circumferential direction of the instrument channel gradually increases.

Optionally, the first edge surface and the second edge surface are each connected to an adjacent portion of the corresponding force-applying block through an arc-shaped transition surface.

Optionally, for each force-applying block, an angle between the first edge surface and the second edge surface in the circumferential direction of the instrument channel is 30 degree.

Optionally, for each force-applying block, an angle between a movement direction of the force-applying block and the second edge surface is 105 degrees, and an angle between the movement direction of the force-applying block and the first edge surface is 75 degrees.

Optionally, in a circumferential direction of the instrument channel, an angle between movement directions of two adjacent force-applying blocks is 30 degrees.

Optionally, the housing is in a shape of a hollow disc, the instrument channel extends through an axis of the disc, an interior of the housing is an installation chamber, and the ring gear and the force-applying blocks are all located in the installation chamber.

The present disclosure further provides a method for loading an interventional instrument, including:
providing a crimper according to any one of the above embodiments;
placing the interventional instrument in the instrument channel;
rotating the ring gear to drive the plurality of force-applying blocks to move synchronously to crimp the interventional instrument; and
transferring the crimped interventional instrument into a sheath for loading the interventional instrument.

Optionally, the sheath is pre-positioned and aligned with the instrument channel.

Optionally, depending on radial sizes of different sections of the interventional instrument, the interventional instrument is crimped by a stepwise crimping method.

Optionally, the interventional instrument includes a first section and a second section in its own axial direction, and a radial size of the first section is larger than that of the second section.

The stepwise crimping method includes:
inserting a mandrel into the interventional instrument and crimping the first section so that the radial size of the first section is approximately equal to the radial size of the second section; and
synchronously crimping the first section and the second section until the first section and the second section are in contact with the mandrel.

Optionally, the method further includes inserting a first sheath of a delivery system into the interventional instrument, crimping the interventional instrument until it is in contact with the first sheath, and pushing the crimped interventional instrument into a second sheath of the delivery system.

The crimper disclosed in the present disclosure drives the force-applying blocks to move through the ring gear, which reduces the number of components of the crimper and can better control the die sinking costs and the sizes of the components, and simplify the assembly, avoiding a cumbersome assembly by fixing.

The present disclosure further provides a crimper for crimping the interventional instrument, including:
a housing which is hollow and has a through instrument channel;
a plurality of force-applying blocks which are movably installed in the housing and distributed around the instrument channel, the plurality of force-applying blocks can be relatively gathered and separated, thereby contracting and expanding the instrument channel; at least one of the force-applying blocks is provided with an indicator, and the housing is provided with a view port corresponding to the indicator.

The structural features and driving mechanism of the plurality of force-applying blocks in this technical solution can either use conventional technologies or refer to other embodiments of the present disclosure. For example, a ring gear and a driving mechanism can be used to synchronously drive the plurality of force-applying blocks.

Optionally, the indicator and the corresponding force-applying block are formed in one or separate pieces, and the indicator is located on one side of the corresponding force-applying block in an axial direction of the housing (that is, in the extension direction of the instrument channel).

Optionally, when the indicator and the corresponding force-applying block are formed in separate pieces, the indicator and the corresponding force-applying block are fixed together by insertion-fit and/or bonding.

Optionally, one of the indicator and the corresponding force-applying block is provided with an insertion portion, and the other is provided with a slot that matches the insertion portion.

Optionally, the view port is elongated and extends in a movement direction of the corresponding force-applying block where the indicator is located.

Optionally, a movement direction of the corresponding force-applying block with the indicator is parallel to a bottom surface of the crimper.

Optionally, the view port is hollowed out of the housing, and a transparent cover is provided at the hollowed out portion.

Optionally, the transparent cover is snapped into the hollowed out portion of the housing.

Optionally, an end of the indicator extends out of the view port to form an indication portion, and a width of the indication portion is greater than a width of the view port.

Optionally, the housing has a recessed area surrounding the view port, and the indication portion is disposed in the recessed area; and
the transparent cover is snapped into the recessed area and is flush with a surrounding portion of the housing.

Optionally, there are at least two indicators, and the at least two indicators are located on two opposite sides of the housing in an axial direction of the housing.

Optionally, there are two indicators which are fixed on the same force-applying block.

Optionally, the indicator has a different color from the housing.

Optionally, the housing and/or the transparent cover are provided with a reference scale for showing displacement of the indicator.

The present disclosure further provides a crimper for crimping the interventional instrument, including:
a housing which is hollow and has a through instrument channel;
a plurality of force-applying blocks which are movably installed in the housing and distributed around the instrument channel, the plurality of force-applying blocks can be relatively gathered and separated, thereby contracting and expanding the instrument channel;
a ring gear rotatably installed in the housing and located around the instrument channel, the ring gear is connected to the force-applying blocks in a transmission manner and configured to synchronously drive the plurality of force-applying blocks; and
a driving shaft, which has at least a part located in the housing and linked with the ring gear and at least a part for connecting a power source.

Optionally, a rotation axis of the driving shaft is parallel to a rotation axis of the ring gear.

Optionally, the power source is a knob and/or a motor;
at least part of the knob is exposed out of the housing, and the knob and the driving shaft are formed in one or separate pieces; and
the motor is located inside the housing.

Optionally, the crimper has opposite front and back surfaces in an extension direction of the instrument channel, the knob is located on the front surface of the housing, and a support is installed on the front surface of the housing.

Optionally, at least part of the knob is located outside the housing, and an elastic washer is pressed between said part and an outer wall of the housing.

Optionally, the elastic washer is annular and surrounds the knob, and wherein one axial side of the elastic washer is in contact with the housing, and the other side is in contact with the knob.

Optionally, the outer wall of the housing has a concave area corresponding to the knob, and the elastic washer is disposed in the concave area.

Optionally, the driving shaft is located outside the ring gear in a radial direction of the ring gear.

Optionally, the driving shaft is configured to directly drive the ring gear or through a linkage component; and
the linkage component uses a gear set or a worm gear for transmission.

Optionally, the gear set includes a plurality of gears that mesh with each other for transmission, and a rotation axis of each gear is parallel to the driving shaft.

Optionally, the number of the gears is 2 to 5.

Optionally, the gear set includes:
a third gear which is coaxially fixed with the driving shaft; and
a fourth gear rotatably matching with the housing, wherein the fourth gear is engaged between the third gear and the ring gear and used for transmission.

Optionally, the ring gear includes an annular portion, and driving teeth are distributed on an outer periphery of the annular portion for meshing with the linkage component.

Optionally, the third gear and the fourth gear are both located in the housing, and in a radial direction of the ring gear, the third gear and the fourth gear are both located outside the ring gear.

Optionally, the fourth gear includes at least a first tooth unit and a second tooth unit arranged coaxially, meshing with the third gear and the driving teeth respectively; and
a tooth thickness ratio between the first tooth unit and the second tooth unit is in a range of 1:0.7 to 1.5.

Optionally, in an axial direction of the ring gear, an outer periphery of the ring gear is partially offset to form an offset area, and the driving teeth are located on the offset area; and
the ring gear has an averted area on a front side of the offset area for accommodating the first tooth unit or the second tooth unit.

The present disclosure further provides a crimper for crimping the interventional instrument, including:
a housing which is hollow and has a through instrument channel;
a plurality of force-applying blocks which are movably installed in the housing and distributed around the instrument channel, the plurality of force-applying blocks can be relatively gathered and separated, thereby contracting and expanding the instrument channel;
a ring gear rotatably installed in the housing and located around the instrument channel, the ring gear is connected to the force-applying blocks in a transmission manner and configured to synchronously drive the plurality of force-applying blocks, and the ring gear is provided with a plurality of limiting structures located at different circumferential positions of the ring gear; and
a shifter movably installed in the housing and having a plurality of working positions, wherein the shifter is configured to cooperate with the corresponding limiting structure at each working position to limit rotation of the ring gear.

Optionally, the limiting structures have different radial positions on the ring gear, and the shifter is configured to move in a radial direction of the ring gear to the corresponding working positions.

Optionally, each limiting structure is a limiting step provided on the ring gear, and the shifter is configured to abut against the corresponding limiting step in a circumferential direction of the ring gear at the corresponding working position.

Optionally, the crimper further includes a driving mechanism for driving the shifter, and the driving mechanism includes:
an operation button, which is rotatably mounted on the housing;
a transmission member, which is linked to the operation button and configured to drive the shifter to switch the working positions; and
an elastic member acting on the shifter to keep the shifter at the corresponding working position.

Optionally, the transmission member is a cam, and a rotation axis of the cam is perpendicular to a movement direction of the shifter.

Optionally, the operation button is located on a front surface of the housing, and the operation button and a view port on the same surface are respectively located on two sides of the instrument channel.

Optionally, the transmission member is a cam, and the shifter at least includes a driving portion that abuts against an outer peripheral surface of the cam, a locking portion for cooperating with the corresponding limiting structure, and a transmission portion connected with the elastic member; and
in an axial direction of the ring gear, the driving portion and the locking portion are located on two opposite sides of the shifter.

Optionally, the elastic member is a compression spring, and the elastic member is pressed between the transmission portion and an inner wall of the housing for applying force on a radially inner side of the shifter.

Optionally, the shifter is block-shaped and is located between the inner wall of the housing and the ring gear.

Optionally, the driving portion faces a radially outer side of the shifter; and
an orientation of the locking portion is perpendicular to an orientation of the shifter.

Optionally, the shifter has a first groove and a second groove on two opposite sides in the axial direction of the ring gear, wherein the first groove accommodates part of the ring gear, the second groove accommodates the cam, and an inner wall of the second groove is the driving portion.

Optionally, the radially inner side of the shifter has a third groove, and a bottom wall of the third groove is the transmission portion; and
the shifter further includes a post fixed in the third groove, and an end of the elastic member surrounds an outside of the post.

Optionally, the shifter has a top surface and an opposing bottom surface, and the locking portion is located on the top surface of the shifter and is located between the first groove and the third groove.

Optionally, the transmission member is a cam, the operation button and the cam are in a transmission fit through a plurality of transmission gears meshed with each other, and a rotation axis of the operation button, a rotation axis of the cam and rotation axes of the transmission gears are parallel to each other; and
depending on transmission sequence, an initial-stage transmission gear is coaxially arranged with the operation button, and a terminal-stage transmission gear is coaxially arranged with the cam.

Optionally, the shifter is located between two adjacent force-applying blocks in a circumferential direction of the instrument channel, and movement path of the shifter avoids the force-applying blocks.

Optionally, the crimper further includes a locking member for locking the shifter at the corresponding working position, and the locking member directly or indirectly interferes with at least one of the following movable members:
the shifter;
the operation button;
the cam; and
the transmission gears.

Optionally, an inner wall of the housing has a plurality of locking slots for cooperating with the locking member, and the plurality of locking slots are distributed in a rotation or movement direction of the corresponding movable member in sequence.

Optionally, the locking member includes:
a locking tongue for cooperating with the corresponding locking slot; and
a connecting arm connected between the locking tongue and the corresponding movable member and being deformable.

Optionally, the connecting arm extends in an arc shape, two ends of the connecting arm are connected to the corresponding movable member respectively, and the locking tongue is located at a top of the arc of the connecting arm.

Optionally, the crimper further includes an elastic piece installed on the housing; and
the ring gear is provided with a plurality of blocking teeth in its own circumferential direction for cooperating with the elastic piece, and the elastic piece is configured to move over the blocking teeth and vibrate or make sounds.

Specific benefits will be further explained in combination with specific structures or steps in the specific embodiments.

### BRIEF DESCRIPTION OF DRAWINGS

FIG. 1 is a schematic perspective view of a crimper according to an embodiment of the present disclosure;
FIG. 2 is another view of the crimper in FIG. 1 from another perspective;
FIG. 3 is a schematic exploded view of the crimper in FIG. 1;
FIG. 4 is a schematic exploded view of a part of the crimper in FIG. 1;
FIG. 5 is a schematic view of FIG. 4 with the transparent cover removed;
FIG. 6 is a schematic view of an indicator and a force-applying block in FIG. 3;
FIG. 7 is a schematic exploded view of the indicator and the force-applying block in FIG. 6;
FIG. 8 is a schematic view of the crimper in FIG. 1 with part of the housing removed;
FIG. 9 is a schematic view of part of the housing in FIG. 8;
FIG. 10 is a partial cross-sectional view of the housing in FIG. 9;
FIG. 11 is a schematic view of the force-applying blocks and the ring gear in FIG. 8;
FIG. 12 is a schematic view of the force-applying blocks and the ring gear in FIG. 8;
FIG. 13 is a schematic view of the ring gear in FIG. 11;
FIG. 14 is a schematic view of the force-applying blocks and the transmission mechanisms in FIG. 11;
FIG. 15 is a schematic view of the transmission mechanism in FIG. 14;
FIG. 16 is an exploded view of the transmission mechanism in FIG. 15;
FIG. 17 is a schematic view of a part of the crimper in FIG. 1;
FIG. 18 is a schematic exploded view of the knob and the rotation shaft in FIG. 17;
FIG. 19 is a schematic view of a crimper according to an embodiment of the present disclosure;
FIG. 20 is schematic view of the shifter and a part of the housing in FIG. 19;
FIG. 21 is a schematic view of the housing shown in FIG. 20;
FIG. 22 is a schematic view of the shifter and the ring gear;
FIG. 23 is a schematic view of the shifter and the ring gear from another perspective;
FIG. 24 is a schematic view of the shifter in FIG. 20;
FIG. 25 is a schematic view of the first transmission gear in FIG. 22;
FIG. 26 is a schematic view of the second transmission gear in FIG. 22;
FIG. 27 is an exploded view of the crimper and the support in FIG. 19;
FIG. 28 is an exploded view of the crimper and the support in FIG. 27;
FIG. 29 is an exploded view of the support in FIG. 27;
FIG. 30 is a schematic view of the first semi-cylinder of the support in FIG. 29;
FIG. 31 is a schematic structural view of the second semi-cylinder of the support in FIG. 29;
FIG. 32 is an exploded view of the housing and base of the crimper;
FIG. 33 is a schematic view of the base in FIG. 32;
FIG. 34 is a schematic view of an interventional instrument according to an embodiment of the present disclosure;
FIG. 35 is a schematic view of the interventional instrument in FIG. 34 After being compressed;
FIG. 36 is a schematic view of a crimper according to an embodiment of the present disclosure;
FIG. 37 is a schematic view of a crimper according to an embodiment of the present disclosure;
FIG. 38 is a schematic view of the crimper in FIG. 36 with part of the housing removed;
FIG. 39 is a schematic view of the crimper in FIG. 36 with part of the housing removed;
FIG. 40 is a schematic exploded view of the crimper in FIG. 36;
FIG. 41 is a schematic view of the ring gear and force-applying blocks in FIG. 38;
FIG. 42 is a schematic view of the ring gear and force-applying blocks in FIG. 38;
FIG. 43 is a schematic view of the ring gear in FIG. 41;
FIG. 44 is a schematic view showing the transmission mechanisms in FIG. 41;
FIG. 45 is a schematic view showing the transmission mechanisms in FIG. 42;
FIG. 46 is a schematic view of the force-applying block in FIG. 41;
FIG. 47 is a schematic view showing the connection between the driving handle and the housing;
FIG. 48 is a schematic exploded view of the driving handle and housing in FIG. 47;
FIG. 49 is a schematic exploded view of the housing in FIG. 36;
FIG. 50 is a view of a part of the housing in FIG. 49;
FIG. 51 is a schematic view of the housing in FIG. 49;
FIG. 52 is a schematic view of a crimper;
FIG. 53 is a schematic view of a crimper;
FIG. 54 is a schematic exploded view of the crimper in FIG. 53;
FIG. 55 is a flow chart of a method for loading an interventional instrument according to an embodiment of the present disclosure;
FIG. 56 is a schematic view of an interventional instrument;
FIG. 57 is a schematic view of an interventional instrument; and
FIG. 58 is a schematic view showing the interventional instrument being loaded into the delivery system.

### Reference list:

100, crimper; 101, front surface; 102, front surface;
10, housing; 11, instrument channel; 12, first housing half; 121, second housing half; 122, decorative plate; 123, concave area; 13, window; 14, view port; 141, transparent cover; 142, the first snapping block; 143, the first snapping slot; 144, recessed area; 15, mounting column; 151, shaft hole; 152, inner cylinder; 153, outer cylinder; 154, sealing plate; 16, fixing column; 17, installation area; 18, fixing portion; 181, snapping tongue; 182, cantilever arm; 19, guide groove; 191, enlarged opening; 192, guide plate; 193, avoiding area; 194, guide groove;
20, force-applying block; 21, slot; 22, bent portion; 221, first edge surface; 222, second edge surface; 223, arc-shaped transition surface;
30, indicator; 31, insertion portion; 32, indication portion;
40, ring gear; 41, annular portion; 411, driving tooth; 42, inner tooth; 43, transmission mechanism; 431, rack; 432, transmission gear; 433, fifth gear; 434, sixth gear; 435, rotation shaft; 436, second snapping block; 437, second snapping slot; 438, through hole; 44, blocking tooth; 441, first guide tooth surface; 442, second guide tooth surface; 45, limiting structure; 451, limiting step; 452, transition surface; 46, offset area; 47, averted area;
50, driving shaft; 51, power source; 52, knob; 521, fitting groove; 522, shell; 523, connector; 524, third snapping block; 525, third snapping slot; 526, depression; 53, linkage component; 531, third gear; 532, fourth gear; 533, first tooth unit; 534, second tooth unit; 535, axle; 536, axle; 54, first alignment mark;
60, shifter; 61, driving mechanism; 611, operation button; 612, cam; 613, elastic member; 62, transmission gear; 621, first transmission gear; 622, second transmission gear; 63, locking member; 631, locking tongue; 632, locking slot; 633, connecting arm; 64, second alignment mark; 65, driving portion; 651, locking portion; 652, transmission portion; 653, first groove; 654, second groove; 655, third groove; 656, post;
70, elastic piece; 71, connection portion; 72, trigger portion;
80, support; 81, first semi-cylinder; 811, second semi-cylinder; 812, support channel; 813, rotation groove; 814, limiting tooth; 815, fixed shaft; 816, snapping block; 817, operation ear; 818, guide surface; 819, locking surface; 82, elastic liner; 83, attachment portion; 831, snapping block; 832, snapping slot; 84, transition section;
90, base; 91, boss; 911, installation groove; 912, locking slot; 92, positioning component; 921, fool-proof ridge; 922, fool-proof groove; 923, enlarged opening;
200, interventional instrument; 201, stent;
9100, crimper; 9101, instrument channel; 9103, delivery system; 9104, first sheath; 9105, second sheath; 9106, guide head; 9107, installation head;
910, housing; 911, operation window; 912, installation chamber; 913, guide unit; 9131, guide plate; 9132, guide groove; 9133, averted area; 914, window; 915, main body; 916, cover;
920, force-applying block; 921, bent portion; 9211, first edge surface; 9212, second edge surface; 9213, arc-shaped transition surface;
930, ring gear; 931, annular portion; 932, driving teeth; 933, weight-reducing groove; 934, connection portion; 935, snapping slot;
940, transmission mechanism; 941, rack; 942, transmission gear; 9421, first gear; 9422, second gear;
950, driving mechanism; 951, driving handle; 9511, holding portion; 9512, snapping portion; 952, shielding plate;
960, base; 961, snap-fit connection structure; 9611, snapping tongue; 9612, snapping slot; 962, installation groove;
970, limiting component; 971, fixed part; 972, adjustable part; 9721, support groove; 973, locking mechanism; 9731, stud; 9732, operation portion; 9733, threaded hole;
980, support; 981, support channel; 9811, snapping block; 982, elastic liner; 9821, groove; 983, bear portion; 984, support portion; 985, magnetic piece; 986, matching piece; 987, attachment portion; 988, flared section; 989, semi-cylinder;
990, interventional instrument; 991, stent; 992, connecting ear; 993, first section; 994, second section,

### DDESCRIPTION OF EMBODIMENTS

The technical solutions according to the embodiments of the present disclosure will be described clearly and fully in combination with the drawings according to the embodiments of the present disclosure. Obviously, the described embodiments are not all embodiments of the present disclosure, but only part of the embodiments of the present disclosure. Based on the disclosed embodiments, all other embodiments obtained by those skilled in the art without creative work fall into the scope of this invention.

It should be noted that, when a component is "connected" with another component, it may be directly connected to another component or may be indirectly connected to another component through a further component. When a component is "provided" on another component, it may be directly provided on another component or may be provided on another component through a further component.

Unless otherwise defined, all technical and scientific terms used herein have the same meaning as commonly understood by a person skilled in the art. The terms in the description of the present disclosure are used to describe specific embodiments, and not to limit the present disclosure. The term "and/or" used herein includes one or more of the listed options in any combinations, or the combination of all of the listed options.

In the present disclosure, the terms "first", "second" and the like are used for descriptive purposes only and are not to be understood as indicating or implying the relative importance or the number or order of the technical features referred. Thus, features defined with "first", "second" can explicitly or implicitly include one or more of such features. In the description of the present invention, "plurality" means at least two, such as two, three, etc., unless explicitly and specifically defined otherwise.

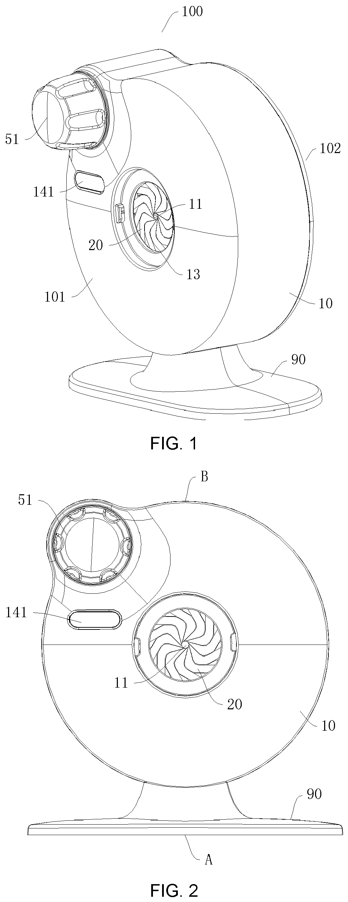

Referring to FIGS. 1 to 3, the present disclosure discloses a crimper 100 for crimping an interventional instrument 200, including:
a housing 10, which is hollow and has a through instrument channel 11; and
a plurality of force-applying blocks 20, which are movably installed in the housing 10 and distributed around the instrument channel 11. The plurality of force-applying blocks 20 can be relatively gathered and separated, thereby contracting and expanding the instrument channel.

In order to facilitate delivery within the human body, the interventional instrument 200 needs to be radially compressed by the crimper 100 before surgery to obtain a smaller radial size. After being compressed, it is loaded into a delivery system and delivered to the treatment site in the human body in a compressed state, and then expands to a functional size at the desired site.

Before the interventional instrument 200 enters the instrument channel 11, the plurality of force-applying blocks 20 are in the separated state. After the interventional instrument 200 enters the instrument channel 11, the plurality of force-applying blocks 20 gradually switch from the separated state to the gathered state, during which, the plurality of force-applying blocks 20 contract the instrument channel 11, and the wall of the instrument channel 11 presses the interventional instrument 200 and uniformly reduces the size of the interventional instrument 200.

The force-applying blocks 20 have the same shape. Therefore, during the installation of the force-applying blocks 20, it is not necessary to identify the types of the force-applying blocks 20, which simplifies the assembly of the force-applying blocks 20.

The crimper 100 has a front surface 101 and an opposing back surface 102. The crimper 100 has a bottom surface (e.g., depicted as A in FIG. 2) that matches a support table (e.g., a work top), and a top surface (e.g., depicted as B in FIG. 2) opposing to the bottom surface. The two ends of the instrument channel 11 are respectively open to the front surface 101 and the back surface 102 of the crimper 100.

An installation chamber is defined inside the housing 10, and the force-applying blocks 20 are movably installed in the installation chamber. The shape of the housing 10 is not strictly limited. For example, the housing 10 in the figure is generally cylindrical. In order to facilitate the assembly of other components, it uses a two-part structure. That is, the housing 10 includes two housing halves that are snapped into each other. Alternatively, in order to facilitate local maintenance or operation, it can be consisted of more parts. In order to fix the two housing halves together, snaps or screws or the like can be used.

The housing 10 has an axial direction in space, and the two ends of the housing 10 in the axial direction correspond to the front surface 101 and the back surface 102 of the crimper 100 respectively. The instrument channel 11 extends in the axial direction of the housing 10.

Each housing half is provided with a respective window 13, and each window 13 is communicated with the installation chamber. The two axial ends of the instrument channel 11 are open to the two windows 13 respectively. When the force-applying blocks 20 crimp the interventional instrument 200, the force-applying blocks 20 are exposed to the respective windows 13.

The wall of the instrument channel 11 is formed by the force-applying blocks 20 that cooperate with each other. It should be noted that the gathered and separated states of the plurality of force-applying blocks 20 are relative concepts. The separated state refers to the state in which the force-applying blocks 20 tend to move radially outwardly and away from each other. When the force-applying blocks 20 are in the gathered state, they crimp the interventional instrument 200.

The instrument channel 11 has a central axis that passes through the geometric center of the instrument channel 11. During contracting and expanding the instrument channel 11 by the force-applying blocks 20, the position of the central axis of the instrument channel 11 is not changed to avoid affecting the crimping of the interventional instrument 200.

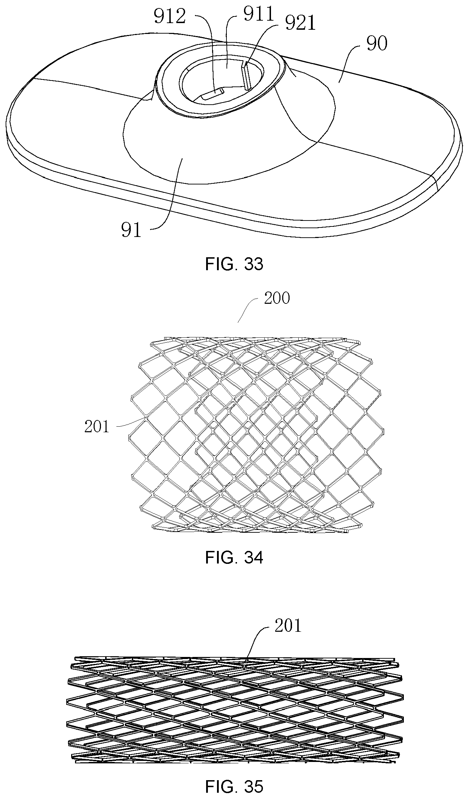

As shown in FIGS. 34 to 35, the interventional instrument 200 (for example, a heart valve prosthesis, while only the stent thereof is shown in the figure) is taken as an example. The interventional instrument 200 includes a cylindrical stent 201. The stent 201 can generally be formed by cutting or braiding. It usually has a meshed structure so that it can be radially deformed. The stent 201 is provided with leaflets (usually two or three, which cooperate with each other to control blood flow), and can be provided with covering film(s) on the inner or outer wall thereof.

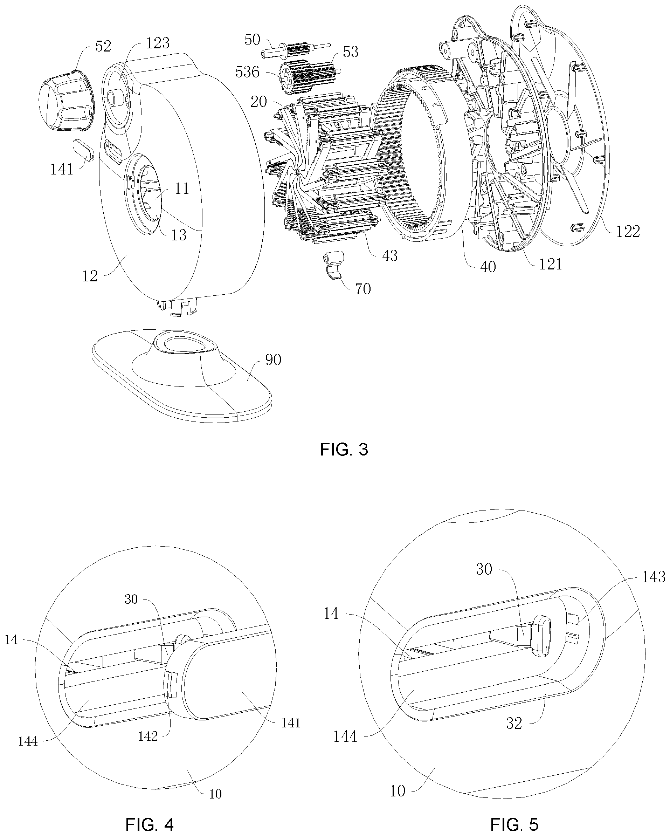

In this embodiment, as shown in FIGS. 4 to 7, a crimper 100 which can indicate the crimping degree of the interventional instrument 200 is provided, including:
a housing 10 which is hollow and has a through instrument channel 11;
a plurality of force-applying blocks 20 which are movably installed in the housing 10 and distributed around the instrument channel 11. The plurality of force-applying blocks 20 can be relatively gathered and separated, thereby contracting and expanding the instrument channel 11; and
at least one of the force-applying blocks 20 is provided with an indicator 30, and the housing 10 is provided with a view port 14 corresponding to the indicator 30 in position.

The indicator 30 can indicate the position of the force-applying blocks 20. During the movement of the force-applying blocks 20, the indicator 30 will be driven to move. By observing the position of the indicator 30 through the view port 14 to determine the movement position of the force-applying blocks 20, the contraction and expansion degree of the instrument channel 11 can be easily determined.

The specific shape of the indicator 30 is not strictly limited (for example, the indicator 30 in the figure is rod-shaped), provided that it is convenient for the operator to observe. In various embodiments, the indicator 30 can protrude from the outer surface of the force-applying block 20, or the indicator 30 can be a part of the force-applying block 20 and is different from the other part of the force-applying block 20 only in color.

In the axial direction of the housing 10, the indicator 30 is located on one side of the force-applying block 20, and the view port 14 is located on the corresponding side of the housing 10, so as to shorten the distance between the view port 14 and the indicator 30. The view port 14 is elongated and extends in the movement direction of the force-applying block 20 where the indicator 30 is located.

In this embodiment, the indicator 30 and the force-applying block 20 are formed in two pieces and fixed with each other by insertion-fit and/or bonding. Alternatively, in other embodiments, the indicator 30 and the force-applying block 20 can be formed in one piece.

In case of insertion-fit, one of the indicator 30 and the force-applying block 20 is provided with an insertion portion 31, and the other is provided with a slot 21 that fits with the insertion portion 31. For example, as shown in the figure, the insertion portion 31 is cylindrical and is fixed on one end of the indicator 30 facing the force-applying block 20. The side wall of the force-applying block 20 is provided with a slot 21 that is substantially in a form-fit with the insertion portion 31.

The movement direction of the force-applying block 20 with the indicator 30 is parallel to the bottom surface of the crimper 100. The bottom surface is generally horizontal during the operation. Compared with a tilt movement, a horizontal movement of the indicator 30 is beneficial to eliminating visual errors.

The view port 14 is hollowed out of the housing 10, and a transparent cover 141 is snapped into the hollowed out portion. For example, the transparent cover 141 is provided with a first snapping block 142, and the housing 10 is provided with a first snapping slot 143 that fits with the first snapping block 142. The transparent cover 141 can seal the view port 14 without interfering the observation of the indicator 30. The housing 10 has a recessed area 144 surrounding the view port 14, and the indication portion 32 is disposed in the recessed area 144. The transparent cover 141 is snapped into the recessed area 144 and is at the same height as the surrounding portion of the housing 10. That is, the outer surface of the transparent cover 141 is roughly flush with the outer surface of the housing 10.

The housing 10 and/or the transparent cover 141 can be provided with reference scales for obtaining the displacement of the indicator 30. The reference scales are distributed sequentially in the movement direction of the indicator 30 and are adjacent to the indicator 30 for comparison.

The end of the indicator 30 extends out of the view port 14 to form the indication portion 32. The width of the indication portion 32 is greater than the width of the view port 14 (in the height direction in FIG. 2), which can prevent the indication portion 32 from falling off and make the indication portion 32 conspicuous. The indication portion 32 and the housing 10 can be configured with different colors for easy differentiation.

A plurality of indicators 30 can be provided, for example, two indicators 30 can be provided respectively on two opposite sides of the housing 10 in the axial direction. The operator can observe the indicators 30 from different sides of the crimper 100. Two indicators 30 can also be fixed on the same force-applying block 20 to ensure the accuracy of indication.

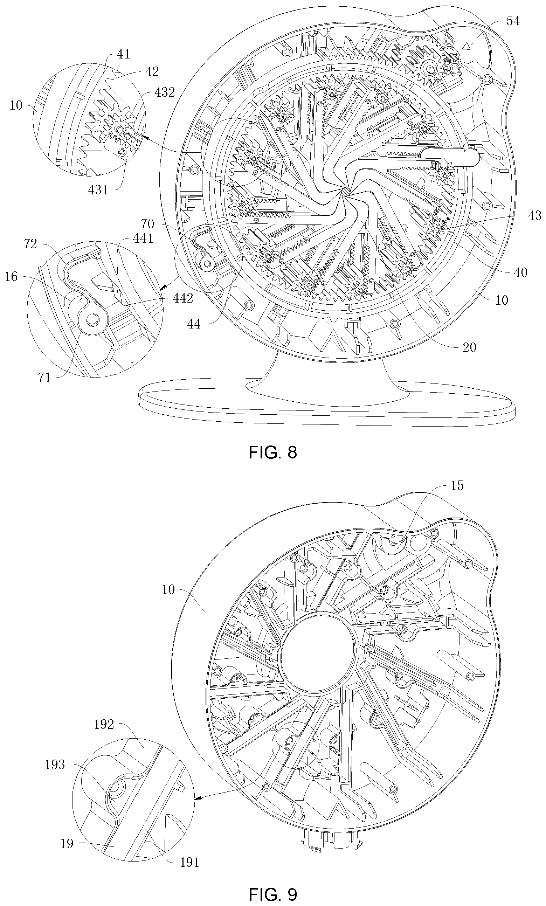

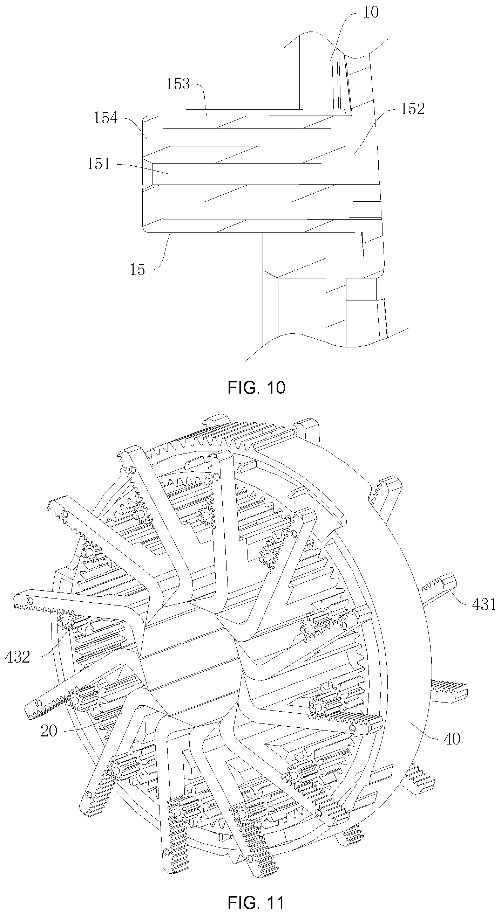

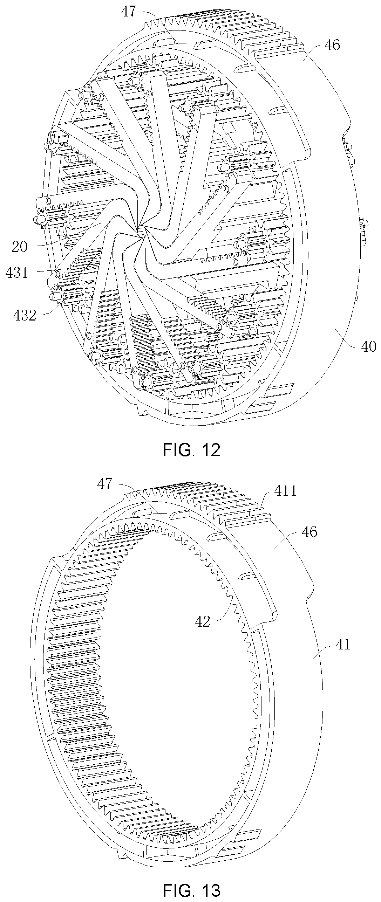

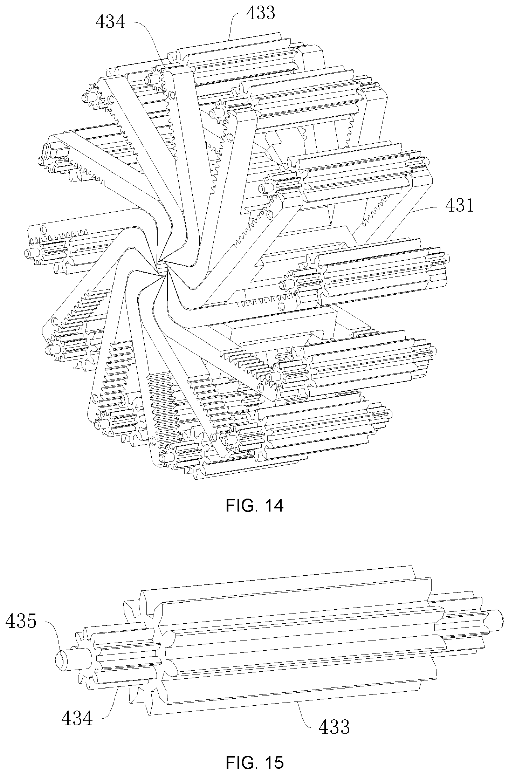

The force-applying blocks 20 can be driven in various ways. In one embodiment, an improved way is provided. As shown in FIGS. 8 to 16, the crimper 100 further includes a ring gear 40, and the ring gear 40 is rotatably engaged with the housing 10. The ring gear 40 can drive the plurality of force-applying blocks 20 synchronously.

The ring gear 40 is rotated to drive the plurality of force-applying blocks 20 to be gathered together and separated from each other. The synchronous movement means that the force-applying blocks 20 move at the same time and at the same speed.

By using the ring gear 40 to drive the force-applying blocks 20 to move, the number of components of the crimper 100 can be reduced, which can better control the die sinking costs and the sizes of the components, and simplify the assembly, avoiding a cumbersome assembly by fixing.

The rotation axis of the ring gear 40 is parallel to the extension direction of the instrument channel 11. The rotation axis of the ring gear 40 is perpendicular to the movement direction of the force-applying block 20. The ring gear 40 includes an annular portion 41 (a circular ring as shown in the figure), and inner teeth 42 arranged on the inner periphery of the annular portion 41 and in transmission fit with the force-applying blocks 20. The ring gear 40 can synchronously drive the plurality of force-applying blocks 20 by the inner teeth 42 based on meshing transmission, so as to improve the stability and accuracy of the ring gear 40 driving the plurality of force-applying blocks 20. Accordingly, there is a certain space in the annular portion 41 and the annular portion 41 surrounds the outer periphery of the plurality of force-applying blocks 20, making the structure of the crimper 100 more compact and reducing the size of the crimper.

In this embodiment, the ring gear 40 is in transmission fit with the force-applying blocks 20 through transmission mechanisms 43. The transmission mechanism 43 includes a rack 431 and a transmission gear 432. Each force-applying block 20 is provided with a rack 431, and the transmission gear 432 is rotatably installed in the housing 10. Each rack 431 is engaged with the inner teeth 42 on the ring gear 40 through one or more transmission gears 432.

When the ring gear 40 rotates, each rack 431 is synchronously driven to move through the transmission gear 432, so as to drive the force-applying block 20 to move. The ring gear 40, the transmission gears 432 and the racks 431 mesh with each other, so that the ring gear 40 can stably drive the force-applying blocks 20 to move.

The ring gear 40 surrounds the outer periphery of the transmission gears 432. The rotation axis of the transmission gear 432 and the rotation axis of the ring gear 40 are parallel to each other. In the extension direction of the instrument channel 11, the rack 431 and the ring gear 40 are offset from each other to reduce interference and allow the rack 431 to have a larger stroke.

In this embodiment, there are multiple groups of transmission gears 432, with each group corresponding to a force-applying block 20. Each group of transmission gear 432 adopts 1 to 3-stage gear transmission. The initial-stage transmission gear meshes with the ring gear 40, and the terminal-stage transmission gear meshes with the rack 431. The transmission gears 432 have the same shape. Therefore, during the installation of the transmission gears 432, it is not necessary to identify the types of the transmission gears 432, which simplifies the assembly of the transmission gears 432.

Each group of transmission gear 432 includes a fifth gear 433 and a sixth gear 434 coaxially fixed with the fifth gear 433. The fifth gear 433 meshes with the ring gear 40, and the sixth gear 434 meshes with the rack 431 on the corresponding force-applying block 20.

When the force-applying block 20 moves, the force-applying block 20 avoids the fifth gear 433 and the sixth gear 434.

The axial length of the fifth gear 433 is L1, the axial length of the sixth gear 434 is L2, and L1: L2 = (3 to 6): 1. The reference diameter of the fifth gear 433 is D1, the reference diameter of the six gears 434 is D2, and D1: D2 = 1: (0.3 to 0.7), preferably, D1: D2 = 1: (0.4 to 0.6). In this embodiment, two racks 431 are fixed on the same force-applying block 20 and are arranged in parallel. In the extension direction of the instrument channel 11, the racks 431 are located on two sides of the ring gear 40 respectively. The transmission gear 432 drives the two racks 431 to move so as to drive the force-applying block 20 to move. The arrangement of the two racks 431 can increase the force-applying points acting on the force-applying block 20 in the extension direction of the instrument channel 11, so as to stabilize the movement of the force-applying block 20. The slot 21 is defined on one side of rack 431.

For each force-applying block 20, each rack 431 is provided with a respective sixth gear 434. In the extension direction of the instrument channel 11, each group of transmission gear 432 includes two sixth gears 434 which are respectively located on two sides of the fifth gear 433. The sixth gears 434 are at least locally positioned on two sides of the ring gear 40, and the local portions mesh with the respective racks 431.

The transmission gear 432 is rotatably engaged with the housing 10 through a rotation shaft 435. The fifth gear 433 and the sixth gears 434 each have a through hole 438 for the rotation shaft 435 to pass through.

The sixth gears 434 and the fifth gear 433 can be formed in one piece. Alternatively, the fifth gear 433 and at least one sixth gear 434 can be formed in separate pieces to facilitate the demoulding of the transmission gear 432. For example, one of the fifth gear 433 and the sixth gear 434 can be provided with a second snapping block 436 at the end surface thereof, and the other can be provided with a second snapping slot 437 at the end surface thereof that fits with the second snapping block 436. After the second snapping block 436 is disposed within the second snapping slot 437, the connection strength can be further enhanced by bonding or the like. Similar to the sixth gear 434 and the fifth gear 433, the sixth gear 434 and the rotation shaft 435 can be formed in one or separate pieces.

The number of inner teeth 42 is an integer multiple (for example, 8 times) of the number of the force-applying blocks 20, to synchronize the phases of the ring gear 40, the transmission gears 432 and the racks 431 so as to ensure that the force-applying blocks 20 move synchronously. For example, the number of inner teeth 42 is 96, and the number of force-applying blocks 20 is 12; the number of inner teeth 42 is 104, and the number of force-applying blocks 20 is 8.

In order to allow the ring gear 40 in a small stroke to drive the force-applying blocks 20 to move in a stroke as larger as possible, in one embodiment, the transmission ratio between the fifth gear 433 and the ring gear 40 is in the range of 1: (5 to 15), and the transmission ratio between the six gear 434 and the ring gear 40 is in the range of 1: (5 to 15). For example, the number of teeth of the fifth gear 433 and the number of the sixth gear 434 are both 10, and the number of teeth of the ring gear 40 is 96, then the transmission ratio between the fifth gear 433 and the ring gear 40 is 1: 9.6, and the transmission ratio between the sixth gear 434 and the ring gear 40 is 1: 9.6. The number of teeth of the fifth gear 433 and the number of the sixth gear 434 are both 12, and the number of teeth of the ring gear 40 is 104, then the transmission ratio between the fifth gear 433 and the ring gear 40 is 3: 26, and the transmission ratio between the sixth gear 434 and the ring gear 40 is 3: 26.

The fifth gear 433 and the sixth gear 434 can alternatively refer to the first gear 9421 and the second gear 9422 described in the following embodiments.

During contraction and expansion of the instrument channel 11, for the continuity of the instrument channel 11 in the circumferential direction, in this embodiment, two adjacent force-applying blocks 20 are always against each other in the circumferential direction of the instrument channel 11 during the movement of the force-applying blocks 20.

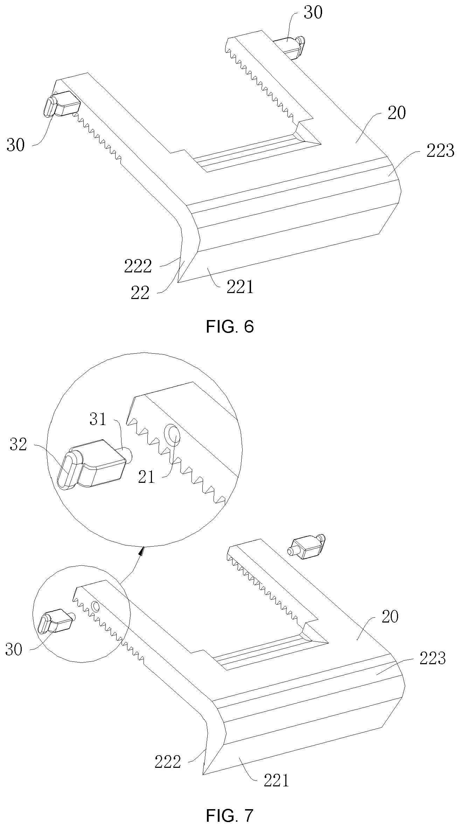

Referring to FIGS. 6 to 7, in this embodiment, one end of the force-applying block 20 facing the instrument channel 11 has a bent portion 22 (roughly hook-shaped). In the circumferential direction of the instrument channel 11, the bent portion 22 has a first edge surface 221 on the outside and a second edge surface 222 on the inside. The wall of the instrument channel 11 is formed by the intersections of the first edge surfaces 221 and the second edge surfaces 222 of the force-applying blocks 20. The rack 431 is connected to the end of the force-applying block 20 facing away from the bent portion 22.

In the circumferential direction of the instrument channel 11, the first edge surface 221 of one of the two adjacent force-applying blocks 20 is in contact with the second edge surface 222 of the other force-applying block 20. In the circumferential direction of the instrument channel 11, the second edge surface 222 of one of the two adjacent force-applying blocks 20 covers a part of the first edge surface 221 of the other force-applying block 20, and the other part of the first edge surface 221 exposed to the instrument channel 11 is served as an action surface. When the force-applying blocks 20 switch from the gathered state to separated state, the length of the action surface in the circumferential direction of the instrument channel 11 gradually increases.

For the same force-applying block, the first edge surface 221 and the second edge surface 222 are each smoothly transitioned to an adjacent portion of the force-applying block 20 through an arc-shaped transition surface 223. The angle between the first edge surface 221 and the second edge surface 222 in the circumferential direction of the instrument channel 11 is 30 degrees. The angle between the movement direction of the force-applying block 20 and the second edge surface 222 is 105 degrees, and the angle between the movement direction of the force-applying block 20 and the first edge surface 221 is 75 degrees. In the circumferential direction of the instrument channel 11, the angle between the movement directions of two adjacent force-applying blocks 20 is 30 degrees.

As shown in FIG. 9, in order to limit the movement of the force-applying blocks 20, in one embodiment, the inner wall of the housing 10 is provided with guide grooves 19. A part of the force-applying block 20 is received in the corresponding guide groove 19 and can slide along the guide groove 19. For example, for the same force-applying block 20, two guide grooves 19 are provided and respectively located at two opposite sides of the force-applying block 20 in the extension direction of the instrument channel 11. The opening of the guide groove 19 faces the force-applying block 20, and includes an enlarged opening 191. The enlarged opening 191 can guide the force-applying block 20 into the guide groove 19. The guide groove 19 is locally enlarged to form an avoiding area 193 for accommodating the sixth gear 434.

In this embodiment, the guide groove 19 is defined between two opposing guide plates 192 on the inner wall of the housing 10, which are formed in one piece with the housing 10. In the circumferential direction of the instrument channel 11, two opposite sides of the force-applying block 20 are respectively in contact with the respective guide plates 192 to prevent the force-applying block 20 from shaking in the circumferential direction of the instrument channel 11.

In this embodiment, as shown in FIG. 8, the crimper 100 further includes an elastic piece 70 installed on the housing 10. The ring gear 40 is provided with a plurality of blocking teeth 44 matching the elastic piece 70 in its circumferential direction. The elastic piece 70 can move over the blocking teeth 44 and then vibrate or make sounds.

One end of the elastic piece 70 is a connection portion 71 fixedly connected to the housing 10, and the other end is a trigger portion 72 that matches the blocking teeth 44. The trigger portion 72 extends to the outer wall of the ring gear 40 and is in clearance fit with the outer wall of the ring gear 40.

The connection portion 71 is generally cylindrical. The inner wall of the housing 10 is provided with a fixing column 16. The connection portion 71 surrounds the fixing column 16 and is fixed to the fixing column 16. The axis of the fixing column 16 is parallel to the axial direction of the housing 10. For example, the outer wall of the fixing column 16 is provided with a snapping block, and the connection portion 71 is provided with a snapping slot that fits with the snapping block.

The trigger portion 72 is sheet-like and bent roughly into a C shape. The trigger portion 72 is fixed on the side wall of the connection portion 71 and is formed in one piece with the connection portion 71. At least the trigger portion 72 of the elastic piece 70 is made of elastic material.

There are a plurality of blocking teeth 44 (for example, three blocking teeth are shown in the figure), and they are arranged on the outer periphery of the ring gear 40. In the circumferential direction of the ring gear 40, two sides of the blocking tooth 44 have a first guide tooth surface 441 and a second guide tooth surface 442 respectively. The first guide tooth surface 441 and the second guide tooth surface 442 meet to form a tooth tip on the side facing away from the ring gear. The trigger portion 72 will vibrate or make a sound when it moves over the tooth tip.

With reference to FIGS. 1 to 3, in this embodiment, the housing 10 includes:
a first housing half 12 and a second housing half 121 snapped into each other, the first housing half 12 and the second housing half 121 enclosing an installation chamber; and
a decorative plate 122 snapped into the first housing half 12 or the second housing half 121 and covering the back surface of the housing 10.

The first housing half 12 is generally cylindrical (for example, like the cylinder shown in the figure). One axial end of the first housing half 12 is closed and the other end is open. The closed end of the first housing half faces the front surface of the housing 10, and the open end faces the back surface of the housing 10.

The second housing half 121 is plate-shaped and is snapped into the opening of the first housing half 12. The second housing half 121 and the first housing half 12 can be connected with each other by screws. The decorative plate 122 is snapped into the second housing half 121.

In this embodiment, the part of the outer periphery of the housing 10 other than the connection of the housing 10 with a base 90 is defined as the middle and upper part, and the outer periphery of the middle and upper part is closed. The components inside the housing 10 do not protrude outward in the radial direction from the middle and upper part. Furthermore, the outer periphery of the middle and upper part extends smoothly to make the appearance of the housing 10 more concise and avoid undesired scratches.

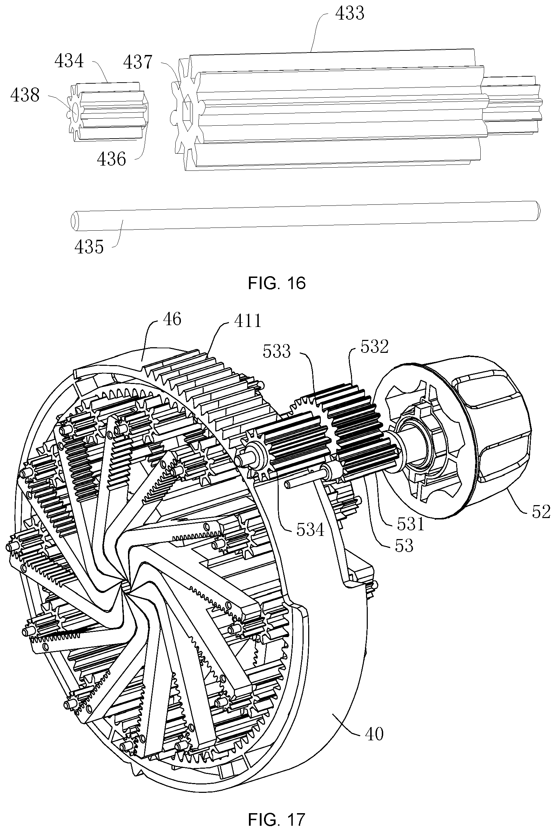

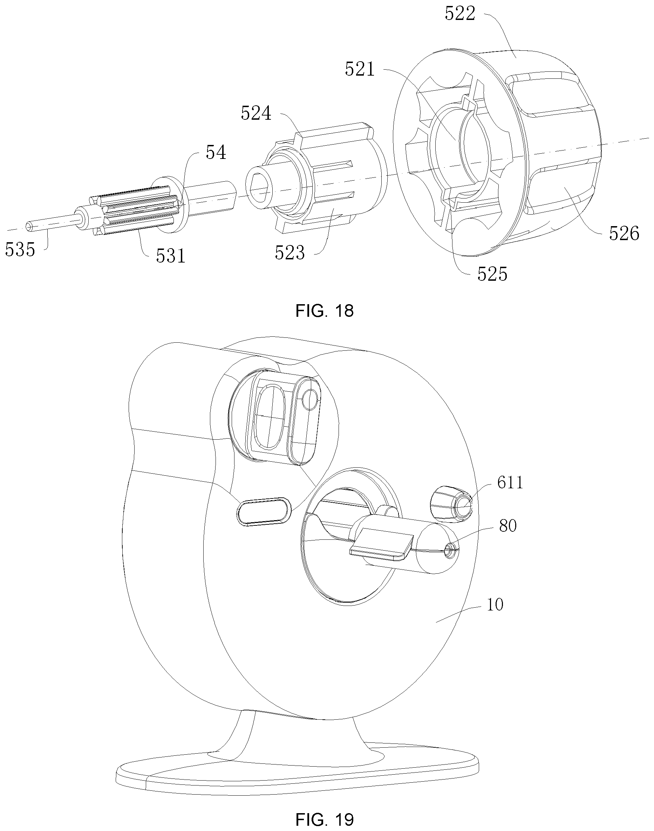

In this embodiment, as shown in FIGS. 17 and 18, a crimper 100 applied to the interventional instrument 200 is provided, including:
a housing 10, which is hollow and has a through instrument channel 11;
a plurality of force-applying blocks 20, which are movably installed in the housing 10 and distributed around the instrument channel 11, and can be relatively gathered and separated, thereby contracting and expanding the instrument channel;
a ring gear 40, which is rotatably installed in the housing 10 and surrounds the instrument channel 11, and is connected to the force-applying blocks 20 in a transmission way and configured to synchronously drive the plurality of force-applying blocks 20; and
a driving shaft 50, which has at least a part located in the housing 10 and linked with the ring gear 40, and at least a part for connecting a power source 51.

The power source 51 provides power for the driving shaft 50. The driving shaft 50 only needs to drive the ring gear 40 to rotate so that the force-applying blocks 20 can be gathered together and separated from each other, thereby reducing the difficulty of alignment of the driving shaft 50 and the ring gear 40 as well as the difficulty of assembling the driving shaft 50 and the ring gear 40.

The driving mechanism of the ring gear 40 for the force-applying blocks 20 and the structure of the housing 10 can refer to the above embodiments.

The driving shaft 50 is a component that can at least transmit torque. The power source 51 can be an electrically-operated component or a manually driven component known in the art. When the motion mode of the power source 51 is inconsistent with the motion mode of the driving shaft 50, appropriate transmission components can be used to transform and transfer the motion. For example, the power source 51 can be a knob 52, at least a part of which is exposed out of the housing 10, and / or a motor which is located inside the housing 10. The following embodiments are explained using the knob 52 as the power source 51.

The knob 52 and the driving shaft 50 can be formed in one or separate pieces. In case of separate pieces, the knob 52 and the driving shaft 50 are directly or indirectly connected with each other. For example, as shown in the figure, the knob 52 has a fitting groove 521, and one axial end of the driving shaft 50 extends into the fitting groove 521 and is engaged with the fitting groove 521, so that the driving shaft 50 can rotate synchronously with the knob 52. In the radial direction of the driving shaft 50, the cross-section of the fitting groove 521 is non-circular, and the outer contour of the driving shaft 50 is substantially the same as the fitting groove 521 to ensure synchronous rotation between the driving shaft 50 and the knob 52.

Regarding the construction of the knob 52, in one embodiment, the knob 52 includes a shell 522 and a connector 523 received in the shell 522 and engaged with the shell 522. The fitting groove 521 is opened in the connector 523. The operator can hold the outer wall of the shell 522 and drives the driving shaft 50 to move through the connector 523.

The shell 522 and the connector 523 are generally cylindrical. One axial end of the shell 522 is closed, and the other end is open. The outer rim of the opening of the shell 522 fits a side wall of the housing 10. One axial end of the connector 523 extends into the shell 522, and the other end extends into the housing 10. For example, the outer wall of the connector 523 can be provided with a third snapping block(s) 524, and the inner wall of the shell 522 can be provided with a third snapping slot(s) 525 that fits with the third snapping block(s) 524, so as to connect the shell 522 and the connector 423. For convenience of holding, the outer periphery of the shell 522 is provided with a plurality of depressions 526.

In this embodiment, in the extension direction of the instrument channel 11, the knob 52 is located on the front surface of the housing 10. At least a part of the knob 52 is located outside the housing 10, and an elastic washer is provided between this part and the outer wall of the housing 10. The elastic washer can increase the friction between the knob 52 and the housing 10 for positioning of the knob 52. The outer wall of the housing has a concave area 123 corresponding to the position of the knob 52. The elastic washer is placed in the concave area 123 and is flush with the surrounding portion of the housing 10. For example, the elastic washer is annular, and surrounds the knob 52 (the connector 523). One axial side of the elastic washer is in contact with the housing 10, and the other side is in contact with the knob 52 (the side of the shell 522 facing the housing 10).

The driving shaft 50 has an axis in space, and the knob 52 can drive the driving shaft 50 to rotate around its own axis, where the axis of the driving shaft 50 can be understood as the rotation axis of the driving shaft 50. The rotation axis of the driving shaft 50 is parallel to the rotation axis of the ring gear 40. In the radial direction of the ring gear 40, the driving shaft 50 is located outside the ring gear 40 to avoid increasing the size of the crimper 100 in the extension direction of the instrument channel 11, so that the structure of the crimper 100 is more compact.

The ring gear 40 is directly driven by the driving shaft 50 or through a linkage component 53. Driving teeth 411 meshing with the linkage component 5 3 are distributed on the outer periphery of the annular portion 41. The housing 10 protrudes radially outward, and the outward protrusion of the housing 10 is transitioned to the other part of the housing 10 through arc surfaces. The outward protrusion can increase the internal space of the housing 10 and facilitate the assembly of the driving shaft 50 and the linkage component 53.

The linkage component 53 transmits the output of the driving shaft 50 to the ring gear 40 to rotate the ring gear 40. According to the installation position and motion mode of the driving shaft 50 and the ring gear 40, the linkage component 53 can use various exiting transmission mechanisms, and can change the direction and speed of movement to adapt to the working characteristics of the driving shaft and ring gear. For example, the linkage component 53 can use a gear set or a worm gear for transmission. The following embodiments are explained using a gear set as the linkage component 53.

The gear set includes a plurality of gears that mesh with each other for transmission, and the rotation axis of each gear is parallel to the driving shaft. The number of gears is 2 to 5. For example, the number of gears as shown in the figure is 2. For example, the gear set includes a third gear 531 and a fourth gear 532. The third gear 531 is coaxially fixed with the driving shaft 50. The fourth gear 532 is rotatably engaged with the housing 10. The fourth gear 532 is engaged between the third gear 531 and ring gear 40 and used for transmission. Both the third gear 531 and the fourth gear 532 are located in the housing, and in the radial direction of the ring gear 40, the third gear 531 and the fourth gear 532 are both located outside the ring gear 40.

The fourth gear 532 includes at least a first tooth unit 533 and a second tooth unit 534 arranged coaxially. The first tooth unit 533 meshes with the third gear 531, and the second tooth unit 534 meshes with the driving teeth 411. The tooth thickness ratio between the first tooth unit 533 and the second tooth unit 534 is 1:0.7 to 1.5, so as to enhance the structural strength between the first tooth unit 533 and the second tooth unit 534. In the axial direction of the ring gear 40, the outer periphery of the ring gear 40 is partially offset to form an offset area 46. The driving teeth 411 are located in the offset area 46. The ring gear 40 has an averted area 47 on the front side of the offset area 46 (facing the front surface of the housing 10) for accommodating the first tooth unit 533. The reference diameter of the first tooth unit 533 is D3, the reference diameter of the second tooth unit 534 is D4, and D3: D4 = 1: (0.4 to 0.7).

In combination with FIGS. 3, 9 and 10, the inner wall of the housing 10 is provided with a mounting column 15. The mounting column 15 has a double-layer structure with one layer inserted in the other. The double-layer structure has a shaft hole 151 inside for matching with an axle 536 of the fourth gear 532. The double-layer structure can strengthen the connection strength between the mounting column 15 and the housing 10 and provide stable support to the axle 536. The mounting column 15 and the housing are formed in one piece.

The mounting column 15 includes an inner cylinder 152, an outer cylinder 153 and a sealing plate 154. The inner cylinder 152 has the shaft hole 151, and the outer cylinder 153 surrounds the outside of the inner cylinder 152. The sealing plate 154 is annular, the inner periphery of the sealing plate 154 is connected to the end of the inner cylinder 152, and the outer periphery of the sealing plate 154 is connected to the end of the outer cylinder 153.

The mounting column 15 further includes reinforcing ribs, which are fixed between the inner cylinder 152 and the outer cylinder 153 and extend in the axial direction of the mounting column 15. The reinforcing ribs are arranged in the axial direction of the inner cylinder 152.

In this embodiment, a first alignment mark 54 is provided between the driving shaft 50 and the housing 10 to indicate the predetermined direction of the knob 52 and to determine the installation position of the knob 52. A pair of first alignment marks 54 is provided, wherein one of the first alignment marks 54 is provided on the inner wall of the housing 10, and the other first alignment mark 54 is provided on the driving shaft 50.

The specific shape of the first alignment mark 54 is not strictly limited. For example, the first alignment mark 54 can be shaped as a triangle, square, etc. The predetermined direction of the knob 52 may be determined through a direction mark provided on the knob 52 or the shape of the knob 52 (for example, the knob 52 is strip-shaped as shown in the figure).

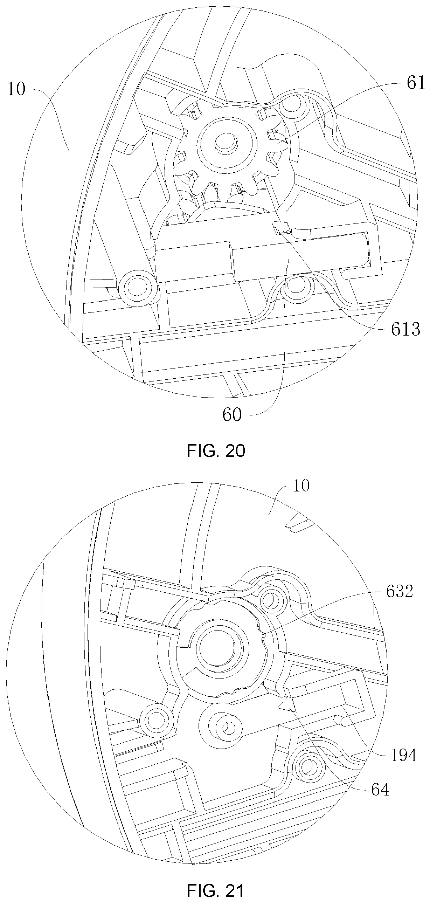

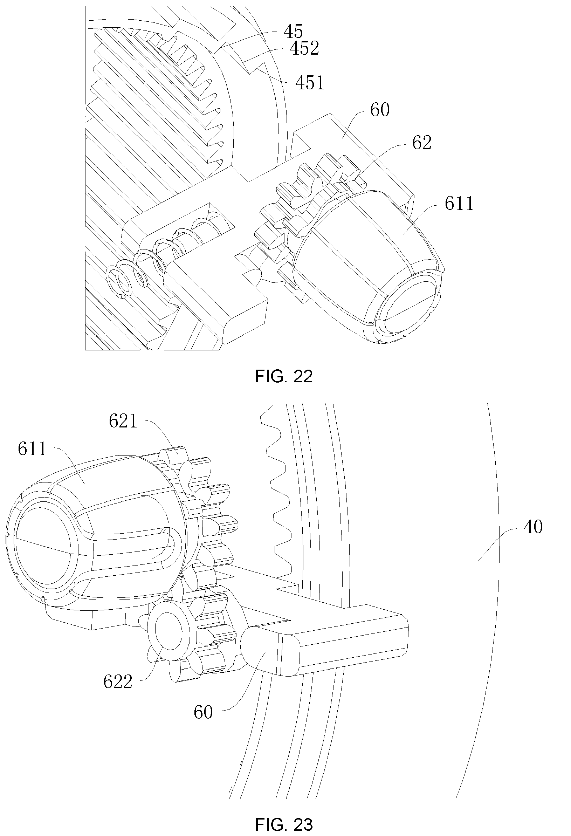

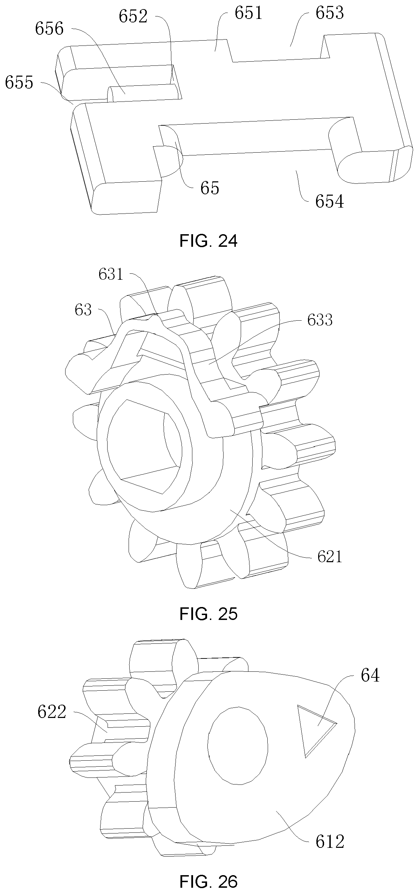

In this embodiment, as shown in FIGS. 19 to 26, a crimper 100 applied to the interventional instrument 200 is provided, including:
a housing 10, which is hollow and has a through instrument channel 11;
a plurality of force-applying blocks 20, which are movably installed in the housing 10 and distributed around the instrument channel 11. The plurality of force-applying blocks 20 can be relatively gathered and separated, thereby contracting and expanding the instrument channel;
a ring gear 40, which is rotatably installed in the housing 10 and surrounds the instrument channel 11, and is connected to the force-applying blocks 20 in a transmission way and configured to synchronously drive the plurality of force-applying blocks 20; the ring gear 40 is provided with a plurality of limiting structures 45 located at different circumferential positions of the ring gear 29; and
a shifter 60, which is movably installed relative to the housing 10 and has a plurality of working positions, and is engaged with the corresponding limiting structure 45 at each working position to limit the rotation of the ring gear 40.

During the rotation of the ring gear 40, the limiting structures 45 rotate synchronously with the ring gear 40. When the shifter 60 is engaged with the corresponding limiting structure 45, the position of the ring gear 40 and thus the position of the force-applying blocks 20 will be fixed, thereby limiting the crimping of the interventional instrument 200 by the force-applying blocks 20. During the rotation of the ring gear 40, the different limiting structures 45 will be engaged with the shifter 60 to crimp the interventional instrument 200 stepwise.

The driving mechanism of the ring gear 40 for the force-applying blocks 20 and the structure of the housing 10 can refer to the above embodiments.

The limiting structures 45 have different radial positions on the ring gear 40. The shifter 60 moves in the radial direction of the ring gear 40 to the corresponding working positions. The limiting structure 45 is a limiting step 451 provided on the ring gear 40. At the working position, the shifter 60 abuts and limits the corresponding limiting step 451 in the circumferential direction of the ring gear 40. The limiting steps 451 are provided along the inner periphery of the ring gear 40 stepwise. There is a transition surface 452 extending in the circumferential direction of the ring gear 40 between two adjacent limiting steps 451. When the shifter 60 moves between two adjacent limiting steps 451, the shifter 60 avoids the transition surface 452, where, in the circumferential direction of the instrument channel 11, the shifter 60 is located between two adjacent force-applying blocks 20, and the movement path of the shifter 60 avoids the force-applying blocks 20.

In this embodiment, the crimper 100 further includes a driving mechanism 61 for driving the shifter 60. The driving mechanism 61 includes an operation button 611, a transmission member and an elastic member 613. The operation button 611 is rotatably mounted on the housing 10, the transmission member is linked with the operation button 611 and can drive the shifter 60 to switch among the working positions, and the elastic member 613 acts on the shifter 60 to keep the shifter 60 at the corresponding working position.

The operation button 611 is located on the front surface of the housing 10. For example, as shown in the figure, the operation button 611 and the view port 14 on the same side are respectively provided on two opposite sides of the instrument channel 11.

The transmission member is a cam 612, and the rotation axis of the cam 612 is perpendicular to the movement direction of the shifter 60. The movement direction of the shifter 60 is parallel to the bottom surface of the crimper 100. The outer peripheral surface of the cam 612 is a continuous curved surface, and the shifter 60 can abut against the outer peripheral surface of the cam 612. When the shifter 60 abuts against the outer peripheral surface of the cam 612 at different positions, the working position of the shifter 60 can be changed.

A second alignment mark 64 is provided between the cam 612 and the housing 10. The second alignment mark 64 is used to indicate the cam 612 making the shifter 60 at the non-working position. The non-working position of the shifter 60 is a position other than the working position, at which position, for example, the outer surface of the cam 612 that is farthest from the rotation axis is in contact with the locking portion 651 of the shifter 60.

A pair of second alignment marks 64 is provided, wherein one second alignment mark 64 is provided on the inner wall of the housing 10, and the other second alignment mark 64 is provided on the side wall of the cam 612. The specific shape of the second alignment mark 64 is not strictly limited. For example, the second alignment mark 64 can be shaped as a triangle, square, etc.

The operation button 611 and the cam 612 are in transmission fit through a plurality of transmission gears 62 meshed with each other, and the rotation axis of the operation button 611, the rotation axis of the cam 612 and the rotation axes of the transmission gears 62 are parallel to each other. For example, as shown in the figure, the number of the transmission gears 62 is two, namely a first transmission gear 621 and a second transmission gear 622. The first transmission gear 621 is coaxially arranged with the operation button 611, and the second transmission gear 622 is coaxially arranged with the cam 612. The first transmission gear 621 and the second transmission gear 622 are engaged with each other for transmission.

In order to limit the movement of the shifter 60, in one embodiment, the inner wall of the housing 10 is provided with a guide groove 194. A part of the shifter 60 is received in the guide groove 194 and can slide along the guide groove 194, so that the movement path of the shifter 60 can be limited. For example, the number of guide grooves 194 is two, and the guide grooves 194 are respectively located on two opposite sides of the shifter 60 in the axial direction of the housing 10.

In this embodiment, the shifter 60 at least includes a driving portion 65 that abuts the outer peripheral surface of the cam 612, a locking portion 651 that cooperates with the limiting structure 45, and a transmission portion 652 connected with the elastic member 613. In the axial direction of the ring gear 40, the driving portion 65 and the locking portion 651 are located on two opposite sides of the shifter 60, wherein the driving portion 65 faces the radial outer side of the shifter 60, and the orientation of the locking portion 651 is perpendicular to the orientation of the shifter 60.

The shifter 60 is generally in the shape of a block (such as a rectangular body as shown in the figure) and has opposite top and bottom surfaces. The shifter 60 is located between the inner wall of the housing 10 and the ring gear 40. The shifter 60 has a first groove 653 and a second groove 654 in the axial direction of the ring gear 40, and a third groove 655 on the radially inner side. The first groove 653 accommodates a part of the ring gear 40. The second groove 654 accommodates the cam 612, and one inner wall of the second groove 654 is the driving portion 65 and has an arc-shaped surface. The bottom wall of the third groove 655 is the transmission portion 652, and the elastic member 613 is pressed against the bottom wall of the third groove 655. The locking portion 651 is located on the top surface of the shifter 60 and between the first groove 653 and the third groove 655.

In this embodiment, the elastic member 613 is a compression spring, and pressed between the transmission portion 652 and the inner wall of the housing 10 (the radially inner wall of the guide groove 194) so as to apply a force on the radially inner side of the shifter 60. A post 656 is provided in the third groove 655, and one end of the elastic member 613 surrounds the outside of the post 656 so as to fix the elastic member 613.

In this embodiment, the crimper 100 further includes a locking member 63 for locking the shifter 60 in a corresponding working position. The locking member 63 directly or indirectly interferes with at least one of the following movable members: the shifter 60, operation button 611, cam 612 and transmission gear 62.

The inner wall of the housing 10 has a plurality of locking slots 632 for cooperating with the locking member 63. The plurality of locking slots 632 are distributed in sequence in the rotation or movement direction of the movable member.

The locking member 63 is provided on the first transmission gear 621, and the housing 10 is provided with a plurality of locking slots 632 distributed in the circumferential direction of the first transmission gear 621. For example, as shown in the figure, the number of locking slots 632 is three.

The locking member 63 includes a locking tongue 631 and a connecting arm 633. The connecting arm 633 is connected between the locking tongue 631 and the movable member. The connecting arm 633 extends in an arc shape. The two ends of the connecting arm 633 are both connected with the movable member, and the locking tongue is located at the top of the arc of the connecting arm. As shown in the figure, two ends of the connecting arm 633 are connected to the first transmission gear 621 respectively. The opening of the arc of the connecting arm 633 faces the rotation axis of the first transmission gear 621. The locking tongue 631 is located at the middle portion of the connecting arm 633 and away from the rotation axis of the first transmission gear 621. During the rotation of the first transmission gear 621, the locking tongue 631 will snap into the corresponding locking slot 632 to limit the rotation of the first transmission gear 621, and the connecting arm 633 can deform to adapt to the process of the locking tongue 631 entering and exiting the corresponding locking slot 632.

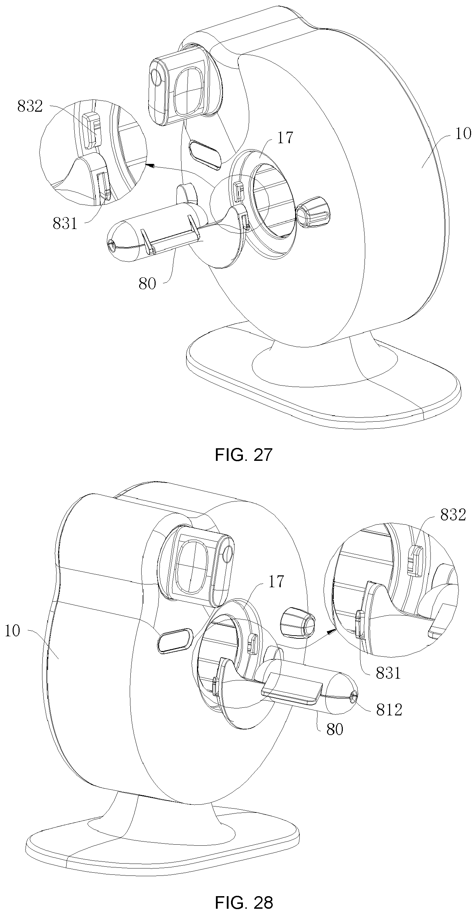

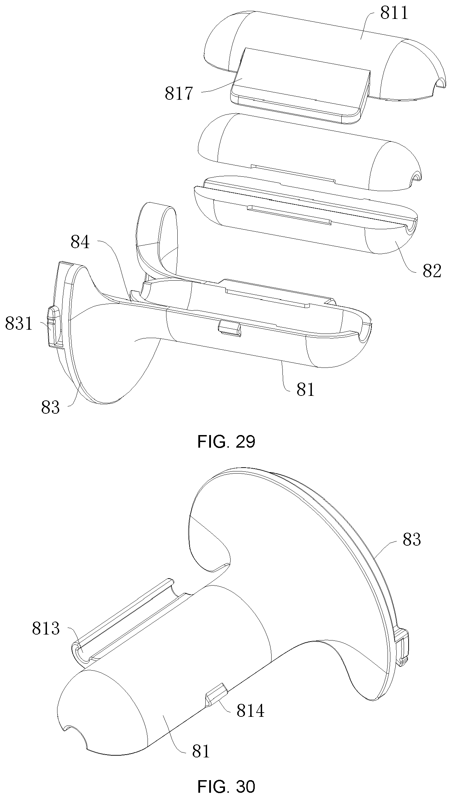

The crimped interventional instrument 200 needs to be loaded into a sheath. In order to support the sheath, in this embodiment, as shown in FIGS. 27 to 31, a support 80 is installed on the front of the housing 10. The support 80 is detachably mounted on the housing 10 in a snapping manner, and has a support position corresponding to the position of the instrument channel 11. When the sheath is placed at the support position, the channel in the sheath faces the instrument channel 11 so that the interventional instrument 200 can enter the channel in the sheath.

The interior of the support 80 serves as a support channel 812. The support 80 has a cylindrical structure. The support 80 includes a first semi-cylinder 81 and a second semi-cylinder 811 that are radially butted together. The space enclosed by the first semi-cylinder 81 and the second semi-cylinder 811 is the support channel 812.

The first semi-cylinder 81 and the second semi-cylinder 811 are snapped into each other. For example, as shown in the figure, the first semi-cylinder 81 is located below the second semi-cylinder 811. One side of the first semi-cylinder 81 has a semi-open rotation groove 813, and the opposite side is provided with a limiting tooth 814. One side of the second semi-cylinder 811 is provided with a fixed shaft 815 engaged with the rotation groove 813, and the opposite side is provided with a snapping block 816 engaged with the limiting tooth 814. The limiting tooth 814 protrudes from the outer wall of the first semi-cylinder 81. The snapping block 816 has a guide surface 818 and a locking surface 819. The limiting tooth 814 moves over the snapping block 816 from the guide surface 818 and abuts against the locking surface 819.

The side wall of the second semi-cylinder 811 is provided with an operation ear 817. The snapping block 816 is provided on the operation ear 817. When the snapping block 816 moves over the limiting tooth 814, the operation ear 817 can deform. The operation ear 817 protrudes outward from the second semi-cylinder 811 via a smooth transition.

The first semi-cylinder 81 is further provided with an attachment portion 83 on a side facing the housing 10. The attachment portion 83 is provided with a plurality of snapping blocks 831, and the housing 10 is provided with a plurality of snapping slots 832 matching the respective snapping blocks 831. For example, as shown in the figure, the number of the snapping blocks 831 is two, and the two snapping blocks 831 are disposed on the attachment portion 83 and located on two radial sides of the instrument channel 11. Correspondingly, the number of the snapping slots 832 is two, and the two snapping slots 832 are located in the housing 10, with different opening directions. When installing the support 80, by rotating the support 80, the snapping blocks 831 can be snapped into the corresponding snapping slots 832, so that the support 80 can be fixed on the housing 10.

The attachment portion 83 is in the shape of a fan ring. The corresponding central angle of the attachment portion 83 is 120 degrees to 180 degrees. The attachment portion 83 corresponds to the lower half of the instrument channel 11. The housing 10 has an installation area 17 surrounding the instrument channel 11. The installation area 17 is recessed. When the attachment portion 83 is placed in the installation area 17, the outer surface of the attachment portion 83 is flush with the outer surface of the housing 10.

The support 80 further includes a transition section 84 fixedly connected between the first semi-cylinder 81 and the attachment portion 83. In the extension direction of the instrument channel 11, the channel in the transition section 84 is gradually enlarged from the first semi-cylinder 81 toward the housing 10 so as to form a large mouth and a small mouth at two ends of the transition section 84. The small mouth of the transition section 84 smoothly transitions to the first semi-cylinder 81, and the large mouth smoothly transitions to the attachment portion 83. The transition section 84 is in the shape of a semi-cylinder. The transition section 84 has a U-shaped cross section in the radial direction of the instrument channel 11. The top of the transition section 84 is open when the crimper 100 is in use, where the top surface of the transition section 84 is substantially flush with the top surface of the first semi-cylinder 81.

An elastic liner 82 is provided in the support channel 812. The elastic liner 82 is installed on the inner wall of the support channel 812. The elastic liner 82 can increase the friction with the sheath. For example, the elastic liner 82 is made of silicone. Alternatively, in other embodiments, the inner wall of the support channel 812 can also be made of silicone, and in this case, the elastic liner 82 can be removed.

The elastic liner 82 has a cylindrical structure, and the side wall of the elastic liner 82 is in contact with the inner wall of the support channel 812. The elastic liner 82 includes two liner units that are radially butted together, and a channel is formed by the two liner units for the sheath to pass through.

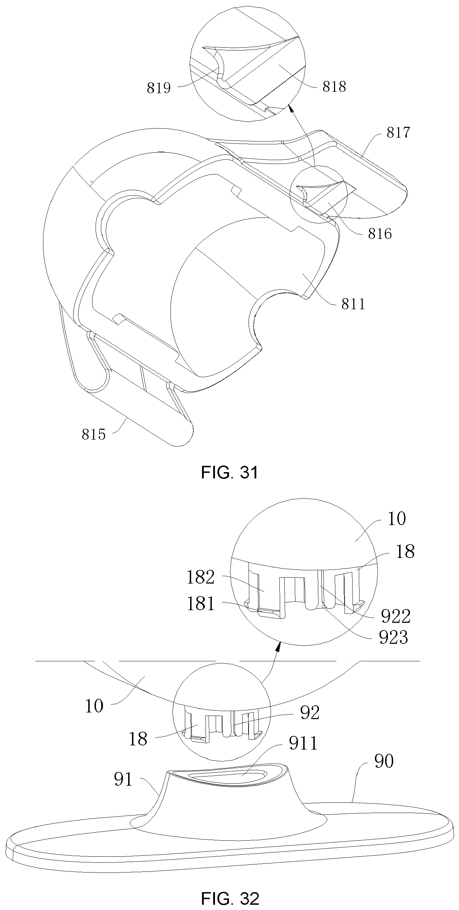

Referring to FIG. 32 and FIG. 33, in this embodiment, the crimper 100 further includes a base 90. The base 90 has an installation groove 911. The outer wall of the housing 10 has a fixing portion 18, and the fixing portion 18 can enter the installation groove 911 so as to fix the housing 10 on the base 90.

The base 90 is generally plate-shaped. The middle portion of the base 90 has an upwardly protruding boss 91, and the installation groove 911 is defined in the boss 91. The top surface of the installation groove 911 is open to the end surface of the boss 91. When the fixing portion 18 is placed in the installation groove 911, the end surface of the boss 91 is in contact with the side surface of the housing 10.

The fixing portion 18 is generally cylindrical and has an axial direction in space. One axial end of the fixing portion 18 is connected to the housing 10 and the other end extends toward the base 90 and is open.

A positioning component 92 is provided between the base 90 and the housing 10 so that the fixing portion 18 and the installation groove 911 have a unique matching relationship in the circumferential direction. The positioning component 92 can determine the installation position relationship between the base 90 and the housing 10. For example, the positioning component 92 includes a fool-proof ridge 921 and a fool-proof groove 922 matching the fool-proof ridge 921. The fool-proof ridge 921 is disposed on one of the groove wall of the installation groove 911 and the side wall of the fixing portion 18, and the fool-proof groove 922 is opened on the other of the groove wall of the installation groove 911 and the side wall of the fixing portion 18.

The unique matching relationship can be understood as: when the fixing portion 18 or the installation groove 911 rotates 360 degrees, there is only one position where the fixing portion 18 and the installation groove 911 match with each other. For example, the number of the fool-proof ridge 921 is one, and the number of the corresponding fool-proof groove 922 is one. The fool-proof groove 922 is provided on the side wall of the fixing portion 18 and extends in the axial direction of the fixing portion 18, and the bottom of the fool-proof groove 922 is open. The bottom of the fool-proof groove 922 has an enlarged opening 923 for guiding the fool-proof ridge 921 to enter.

In order to further strengthen the connection strength between the fixing portion 18 and the installation groove 911, the fixing portion 18 is provided with a snapping tongue 181, and the groove wall of the installation groove 911 is provided with a locking slot 912 that fits with the snapping tongue 181. There is a plurality of snapping tongues 181 extending in the circumferential direction of the fixing portion 18.

Each snapping tongue 181 is connected to the bottom of the fixing portion 18 through a corresponding cantilever arm 182, and the locking slot 912 is located on the bottom wall of the installation groove 911. The cantilever arm 182 extends from the bottom of the fixing portion 18 toward the base 90, and can be deformed to adapt to the installation process of the snapping tongue 181.

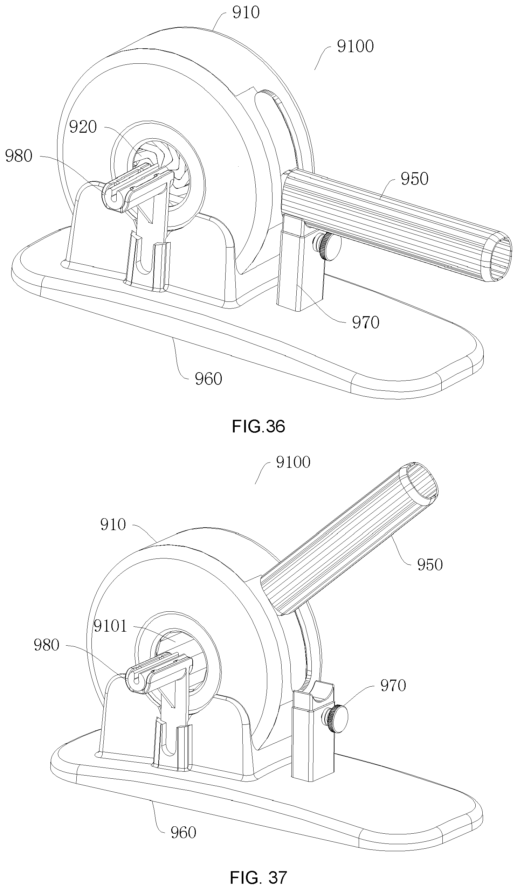

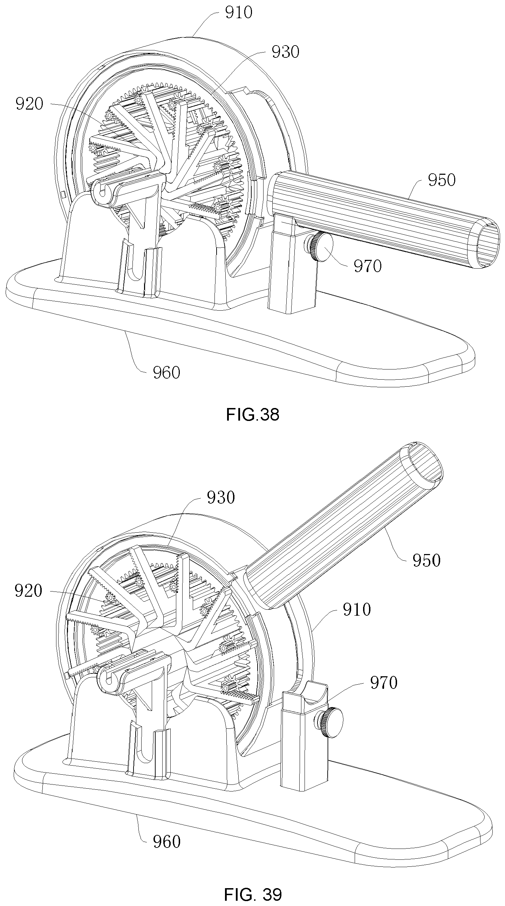

Referring to FIGS. 36 to 40, an embodiment of the present disclosure discloses a crimper 9100 for crimping an interventional instrument, including:
a housing 910 which has a through instrument channel 9101 ;
a plurality of force-applying blocks 920, which are movably installed in the housing 910 and distributed around the instrument channel 9101. The plurality of force-applying blocks 920 have a gathered state (for example, the state of the force-applying blocks 920 shown in FIGS. 38) and a relative separated state (for example, the state of the force-applying blocks 920 shown in FIG. 39), and can contract and expand the instrument channel 9101 during switching the states; and
a ring gear 930 which is rotatably engaged with the housing 910, and can synchronously drive the plurality of force-applying blocks 920 to switch the states.

In order to facilitate delivery within the human body, the interventional instrument 990 needs to be radially compressed by the crimper 9100 before surgery to obtain a smaller radial size. After being compressed, it is loaded into a delivery system and delivered to the treatment site in the human body in a compressed state, and then expands to a functional size at the desired site.

This embodiment further discloses a driving mechanism 950, which includes a driving handle 951 that is indirectly or directly connected to the ring gear 930. The operator can hold the driving handle 951 and pull the driving handle 951 to make the ring gear 930 rotate relative to the housing 910.

Before the interventional instrument 990 enters the instrument channel 9101, the plurality of force-applying blocks 920 are in the separated state. After the interventional instrument 990 enters the instrument channel 9101, the ring gear 930 is rotated to synchronously drive the plurality of force-applying blocks 920 to gradually switch from the separated state to the gathered state. During the state switching process of the plurality of force-applying blocks 920, the plurality of force-applying blocks 920 will contract the instrument channel 9101, and thus the wall of the instrument channel 9101 will press the interventional instrument 990 to uniformly reduce the size of the interventional instrument 990 until the plurality of force-applying blocks 920 transforms into the gathered state. The synchronous movement of the plurality of force-applying blocks 920 means that the force-applying blocks 920 move at the same time with the same movement speed.

The housing 910 is provided with two opposite windows 914 which are both communicated with the installation chamber 912, and the instrument channel 9101 is formed between the two windows 914. When the force-applying blocks 920 are in the gathered state, the force-applying blocks 920 are exposed to the corresponding windows 914.

Here, the force-applying blocks 920 are driven by the ring gear 930 to move, so that the number of components of the crimper 9100 can be reduced, which can better control the die sinking costs and the sizes of the components, and simplify the assembly, avoiding a cumbersome assembly by fixing.

The wall of the instrument channel 9101 is formed by the force-applying blocks 920 that cooperate with each other. It should be noted that the gathered and separated states of the plurality of force-applying blocks 920 are relative concepts. The separated state refers to the state in which the force-applying blocks 920 tend to move radially outwardly and away from each other. When the force-applying blocks 920 are in the gathered state, for example, the diameter of the instrument channel 9101 is at a minimum value. When the force-applying blocks 920 move radially outward from the gathered state, the force-applying blocks 920 are in the separated state.

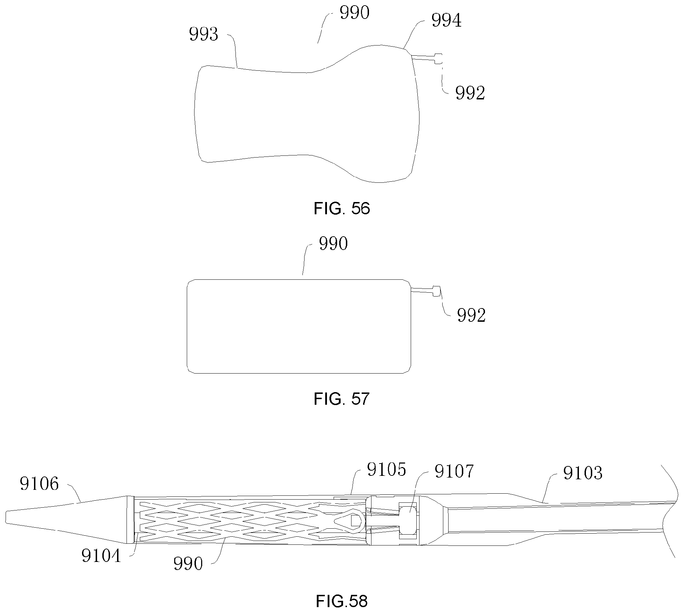

Referring to FIG. 56, the specific shape of the interventional instrument 990 is not strictly limited. For example, the interventional instrument 990 can include a stent 991 with a connecting ear 992 at one axial end of the stent 991. The connecting ear 992 can have an enlarged head at the end. The stent 991 has a radially compressible or expandable structure, generally a meshed cylindrical structure formed by cutting or braiding. In this embodiment, the interventional instrument 990 is a heart valve prosthesis.

In this embodiment, the ring gear 930 synchronously drives the plurality of force-applying blocks 920 to switch between the gathered state and the separated state based on meshing transmission. The meshing transmission can improve the stability and accuracy of the ring gear 930 driving the plurality of force-applying blocks 920. Regarding how the ring gear 930 synchronously drives the plurality of force-applying blocks 920 to switch the states, please refer to the detailed description of the transmission mechanism 940 below, which will not be elaborated here.

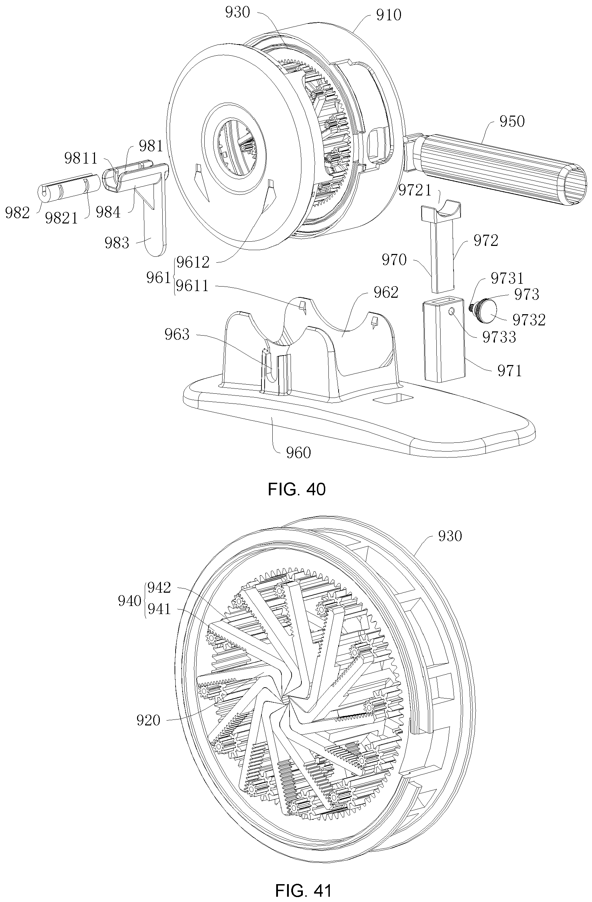

Referring to FIGS. 41 to 46, in this embodiment, the crimper 9100 further includes transmission mechanisms 940, which are connected between the ring gear 930 and the force-applying blocks 920 in a transmission way. The transmission mechanisms 940 are to transmit the output of the ring gear 930 to the force-applying blocks 920 to make the force-applying blocks 920 move synchronously. According to the installation position and motion mode of the ring gear 930 and the force-applying blocks 920, the transmission mechanisms 940 can use various existing transmission mechanisms, and can change the direction and speed of movement to adapt to the working characteristics of the force-applying blocks 920. The transmission mechanism 940 at least provides necessary mechanical strength and good and precise coordination to ensure the desired movement path, speed and reaction time of the force-applying blocks 920.

Referring to FIGS. 41 to 42, in this embodiment, the transmission mechanism 940 includes a rack 941 and a transmission gear 942. The rack 941 is provided on the corresponding force-applying block 920, and the transmission gear 942 is rotatably mounted on in the housing 910. Each rack 941 meshes with the ring gear 930 through one or more transmission gears 942. During the rotation of the ring gear 930, the racks 941 will be synchronously driven by the corresponding transmission gears 942 to move so as to drive the corresponding force-applying blocks 920 to move. Here, the ring gear 930, the transmission gears 942 and the racks 941 mesh with each other, so that the ring gear 930 can stably drive the force-applying blocks 920 to move.

The transmission gear 942 has a rotation axis, which is its own geometric center around which the transmission gear 942 rotates. The ring gear 930 has a rotation axis, which is its own geometric center around which the ring gear 930 rotates.

In this embodiment, multiple groups of transmission gears 942 are provided, and each group corresponds to a force-applying block 920. The transmission gears 942 have the same shape, which facilitates mass production; also, during the installation of the transmission gears 942, there is no need to identify the type of the transmission gears 942, which simplifies the assembly of the transmission gears 942.

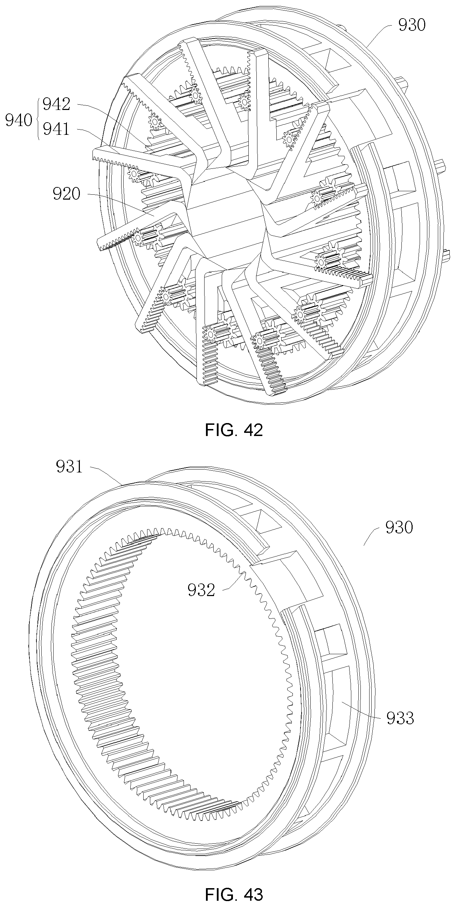

Refer to FIG. 43, for the specific structure of the ring gear 930, in this embodiment, the ring gear 930 includes an annular portion 931, and driving teeth distributed on the annular portion 931 and in transmission fit with the force-applying blocks 920. The driving teeth 932 can be provided in at least one of the following ways:
the driving teeth 932 are inner teeth distributed on the inner periphery of the annular portion;
the driving teeth 932 are outer teeth distributed on the outer periphery of the annular portion 931; and
the driving teeth 932 are side teeth distributed on the axial end surface(s) of the annular portion 931.

In this embodiment, the driving teeth 932 are inner teeth distributed on the inner periphery of the annular portion 931, and the following embodiments will be described on this basis.

In order to reduce the weight of the ring gear 930, in one embodiment, a plurality of weight-reducing grooves 933 are defined in the annular portion 931 in the circumferential direction of the ring gear 930.

There is a certain space in the annular portion 931. In order to make the structure of the crimper 9100 more compact and reduce the size of the crimper 9100, in one embodiment, the ring gear 930 surrounds the plurality of force-applying blocks 920. Also, the ring gear 930 surrounds the transmission gears 942.

In this embodiment, the rotation axis of the ring gear 930 and the extension direction of the instrument channel 9101 are parallel to each other. The instrument channel 9101 has a central axis that passes through the geometric center of the instrument channel 9101. During contracting and expanding the instrument channel 9101 by the force-applying blocks 920, the position of the central axis of the instrument channel 9101 is not changed. The central axis of the instrument channel 9101 substantially coincides with the rotation axis of the ring gear 930 (in this embodiment, the central axis of the instrument channel 9101 coincides with the rotation axis of the ring gear 930). In order to reduce the size of the crimper 9100 in the extension direction of the instrument channel, in one embodiment, the rotation axis of the transmission gear 942 and the rotation axis of the ring gear 930 are parallel to each other.

If the movement path of the rack 941 does not avoid the ring gear 930, the movement path of the force-applying block 920 will be shortened, affecting the contraction and expansion of the instrument channel 9101. In order to enable the movement path of the rack 941 to avoid the ring gear 930, in one embodiment, referring to FIG. 42, in the extension direction of the instrument channel 9101, the rack 941 and the ring gear 930 are offset from each other.

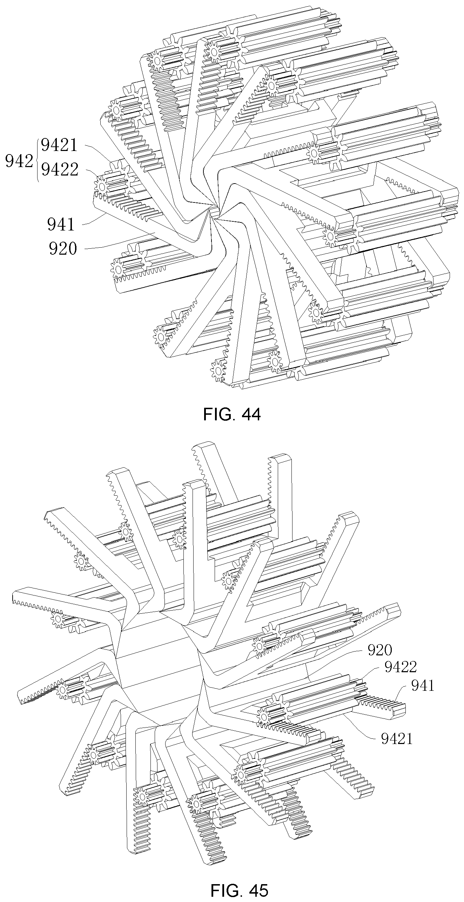

Referring to FIGS. 44 to 46, in this embodiment, two racks 941 are fixed on the same force-applying block 920 in parallel. In the extension direction of the instrument channel 9101, the racks 941 are located on two sides of the ring gear 930 respectively. The transmission gear 942 drives the two racks 941 to drive the force-applying block 920 to move. The arrangement of the two racks 941 can increase the force-applying points acting on the force-applying block 920 in the extension direction of the instrument channel, so as to stabilize the movement of the force-applying block 920.

In this embodiment, the racks 941 and the force-applying block 920 are formed in one piece to increase the structural strength between the racks 941 and the force-applying block 920, while also reducing the difficulty of processing the racks 941 and the force-applying block 920. Alternatively, in other embodiments, the racks 941 and the force-applying block 920 can be formed in separate pieces, and the racks 941 can be fixed to the force-applying block 920 by bonding or welding.

In this embodiment, the number of driving teeth 932 is an integer multiple of the number of force-applying blocks 920 to synchronize the phases of the ring gear 930, the transmission gears 942 and the racks 941 so as to ensure that the force-applying blocks 920 move synchronously. Preferably, the number of the driving teeth 932 is 8 times the number of the force-applying blocks 920. In this embodiment, the number of driving teeth 932 is 96, and the number of force-applying blocks 920 is 12. Alternatively, in other embodiments, the number of driving teeth 932 is 104, and the number of force-applying blocks 920 is 8.

Referring to FIGS. 44 to 45, in this embodiment, the transmission gear 942 adopts 1 to 3-stage gear transmission. The initial-stage gear meshes with the ring gear 930, and the terminal-stage gear meshes with the rack 941. Specifically, in this embodiment, each group of transmission gear 942 includes a first gear 9421 and a second gear 9422 fixedly and coaxially arranged with the first gear 99421. The first gear 9421 meshes with the ring gear 930, and the second gear 9422 meshes with the rack 941 on the corresponding force-applying block 920.

In the radial direction of the instrument channel 9101, when the force-applying blocks 920 are in the gathered state, the ends of the racks 941 away from the force-applying blocks 920 are adjacent to the second gears 9422, and when the force-applying blocks 920 are at the utmost position in the separated state, the racks 941 are close to the inner wall of the housing 910. During the movement of the force-applying blocks 920, the force-applying blocks 920 avoid the first gears 9421 and the second gears 9422 to prevent the first gears 9421 and the second gears 9422 from interfering with the movement of the force-applying blocks 920.

Referring to FIGS. 44 to 46, in this embodiment, for the same force-applying block 920, each rack 941 is configured with a corresponding second gear 9422. In the extension direction of the instrument channel 9101, each group of transmission gear 942 includes two second gears 9422. The two second gears 9422 are respectively located on two sides of the first gear 9421 and are respectively rotatably engaged with the housing 910 through a rotation shaft. In the extension direction of the instrument channel, the two second gears 9422 are at least partially positioned on two sides of the ring gear 930, where the two second gears 9422 mesh with the corresponding racks 941. Alternatively, in other embodiments, for the same force-applying block 920, all racks 941 are configured with the same second gear 9422.

In this embodiment, the axial length of the first gear 9421 is L1, the axial length of the second gear 9422 is L2, and L1: L2 = (3 to 6): 1. The axial length of the first gear 9421 and the axial length of the second gear 9422 are adjusted according to the reference diameter of the first gear 9421, the reference diameter of the second gear 9422 and the circumferential length of the ring gear 930.

As stated above, each force-applying block 920 is configured with a transmission gear 942. In order to avoid mutual interference among the transmission gears 942 when the transmission gears 942 are located in the annular portion 931 of the ring gear 930, there are certain requirements on the reference diameter of the transmission gears 942. In one embodiment, the reference diameter of the first gear 9421 is D1, the reference diameter of the second gear 9422 is D2, and D1: D2 = 1: (0.3 to 0.7). Preferably, D1: D2 = 1: (0.4 to 0.6).

In order to allow the ring gear 930 in a small stroke to drive the force-applying blocks 920 to move in a stroke as larger as possible, in one embodiment, the transmission ratio between the first gear 9421 and the ring gear 930 is in the range of 1: (5 to 15), and the transmission ratio between the second gear 9422 and the ring gear 930 is in the range of 1: (5 to 15). For example, the number of teeth of the first gear 9421 and the number of teeth of the second gear 9422 are both 10, and the number of teeth of the ring gear 930 is 96, then the transmission ratio between the first gear 9421 and the ring gear 930 is 1: 9.6, and the transmission ratio between the second gear 9422 and the ring gear 930 is 1: 9.6. The number of teeth of the first gear 9421 and the number of teeth of the second gear 9422 are both 12, and the number of teeth of the ring gear 930 is 104, then the transmission ratio between the first gear 9421 and the ring gear 30 is 3: 26, and the transmission ratio between the second gear 9422 and the ring gear 930 is 3: 26.

Referring to FIGS. 36 to 40 as well as FIGS. 47 and 48, in this embodiment, the crimper 9100 further includes a driving mechanism 950 for driving the ring gear 930 to rotate. The driving mechanism 950 is mainly used to drive the ring gear 930 to rotate about the center direction of the instrument channel 9101. In order to realize its basic function, the driving mechanism 950 can use a motor, an air cylinder, a hydraulic cylinder or even a manually driven component known in the art. When the motion mode of the driving mechanism 950 is inconsistent with the motion mode of the ring gear 930, appropriate transmission components can be used to transfer and transmit the motion.

Referring to FIGS. 36 to 40 and FIGS. 47 and 48, the specific structure of the driving handle 951 is shown. In one embodiment, the driving handle 951 has a rod-shaped structure, one end of the driving handle 951 is connected to the ring gear 930, and the other end extends in a direction away from the instrument channel 9101.

Regarding the connection between the driving handle 951 and the ring gear 930, in one embodiment, the housing 910 is provided with an operation window 911, and at least a part of the ring gear 930 is exposed to the operation window 911, which part is connected to driving handle 951. The operation window 911 is opposite to the outer periphery of the ring gear 930. The driving handle 951 is connected to the outer periphery of the ring gear 930, and extends away from the ring gear 930. The driving handle extending in the radial direction of the instrument channel 9101 facilitates the operator control on the driving handle 951.

In this embodiment, at least a part of the ring gear 930 is a connection portion 934 exposed to the operation window 911, and the driving handle 951 is detachably fixed to the connection portion 934. The connection portion 934 is mainly used to install the driving handle 951. In order to realize its basic function, the connection portion 934 can be configured as a groove or a protrusion. The driving handle 951 can be fixed to the connection portion 934 by a bolt, a pin, or the like.

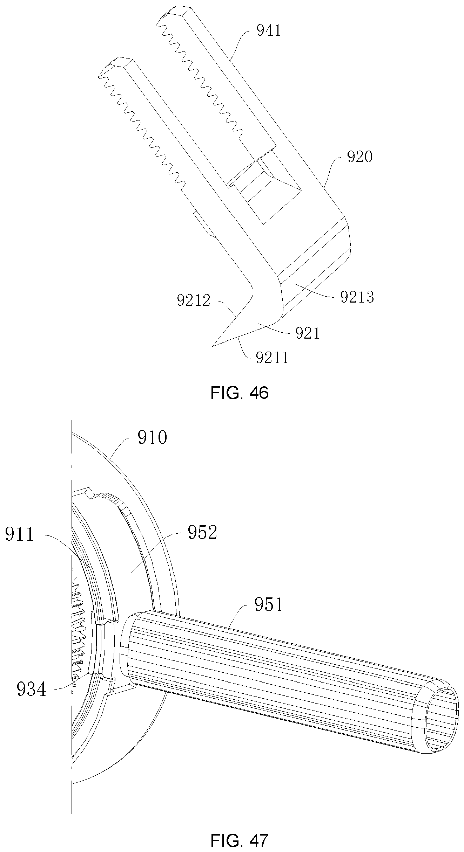

Referring to FIGS. 47 to 48, in this embodiment, the driving handle 951 includes a holding portion 9511 and a snapping portion 9512, and the ring gear 930 is provided with a snapping slot 935 that fits with the snapping portion 9512. The driving handle 951 can be fixed by engaging the snapping portion 9512 into the snapping slot 935.

The snapping portion 9512 is in the shape of a block, and the outer contour of the snapping portion 9512 is substantially consistent with the form of the snapping slot 935, so that the snapping portion 9512 can fit tightly into the snapping slot 935. In the extension direction of the instrument channel 9101, at least one side of the snapping slot 935 is open, so that the snapping portion 9512 can enter the snapping slot 935.

In order to increase the firmness of the snapping portion 9512 in the snapping slot 935, in one embodiment, the driving mechanism 950 further includes a shielding plate 952, and the shielding plate 952 is fixed to the ring gear 930 by snapping, bonding or bolt. The shielding plate 952 and the ring gear 930 cooperate with each other to limit the opening size of the snapping slot 935 facing away from the instrument channel 9101.

In the circumferential direction of the instrument channel, two opposite sides of the operation window 911 can limit the stroke of the driving handle 951. In this embodiment, the central angle corresponding to the stroke of the driving handle 951 is 30 degrees to 120 degrees, and the stroke of the driving handle 951 is determined according to the contraction and expansion degree of the instrument channel. Preferably, the central angle corresponding to the stroke of the driving handle 951 is 30 degrees to 60 degrees. In this embodiment, the central angle corresponding to the stroke of the driving handle 951 is 45 degrees.

Alternatively, in other embodiments, the driving mechanism 950 includes an electrically-operated component connected to the ring gear 930 in a transmission manner. The electrically-operated component can automatically drive the ring gear 930 to rotate to realize the automatic operation of the crimper 9100. Regarding the arrangement of the electrically-operated component, in one embodiment, the electrically-operated component is installed on the housing 910 to drive the ring gear 930 to rotate relative to the housing 910. In this embodiment, the electrically-operated component is a motor.

The motor has an output shaft, and the output shaft is provided with a driving gear. The outer periphery of the annular portion 931 or the axial end surface of the annular portion 931 is provided with driving teeth. The driving mechanism 950 further includes a driving gear meshing with the driving teeth. The electrically-operated component drives the ring gear 930 through the driving gear.

The force-applying blocks 920 cooperate with each other to form the wall of the instrument channel 9101. In order to maintain the continuity of the instrument channel 9101 in the circumferential direction during contraction and expansion of the instrument channel 9101, referring to FIGS. 44 to 46, in the present embodiment, two adjacent force-applying blocks 920 are always against each other in the circumferential direction of the instrument channel 9101 in the process of the force-applying blocks 920 switching from the gathered state to the separated state.

For the specific structure of the force-applying block 920, refer to FIGS. 44 to 46. In this embodiment, one end of the force-applying block 920 facing the instrument channel 9101 has a bent portion 921 (roughly hook-shaped). In the circumferential direction of the instrument channel 9101, the bent portion 921 has a first edge surface 9211 on the outside and a second edge surface 9212 on the inside. The wall of the instrument channel 9101 is formed by the intersections of the first edge surfaces 9211 and the second edge surfaces 9212 of the force-applying blocks 920. The rack 941 is connected to the end of the force-applying block 920 facing away from the bent portion 921. In the extension direction of the instrument channel 9101, two opposite sides of the force-applying block 920 are flush with the outer surfaces of the racks 941 respectively. In the circumferential direction of the instrument channel 9101, the two opposite sides of the force-applying block 920 (excluding the bent portion 921) are flush with the outer surfaces of the racks 941.

Referring to FIGS. 44 to 46, in this embodiment, in the circumferential direction of the instrument channel 9101, for two adjacent force-applying blocks 920, the first edge surface 9211 of one of the force-applying blocks 920 is in contact with the second edge surface 9212 of the other force-applying block 920. Specifically, in one embodiment, in the circumferential direction of the instrument channel 9101, for two adjacent force-applying blocks 920, the second edge surface 9212 of one of the force-applying blocks 920 covers a part of a first edge surface 9211 of the other force-applying block 920, and the other part of the first edge surface 9211 of the other force-applying block 920 exposed to the instrument channel 9101 is the action surface. When the force-applying blocks 920 switch from the gathered state to the separated state, the length of the action surface in the circumferential direction of the instrument channel 9101 gradually increases.

In this embodiment, the first edge surface 9211 and the second edge surface 9212 are each connected to an adjacent portion of the force-applying block 920 through an arc-shaped transition surface 9213, so that the first edge surface 9211 and the second edge surface 9212 can smoothly transition to the adjacent portions of the force-applying block 920 respectively.

Further, regarding the specific construction of the force-applying block 920, in one embodiment, for the same force-applying block 920, the angle between the first edge surface 9211 and the second edge surface 9212 in the circumferential direction of the instrument channel 9101 is 30 degrees. For the same force-applying block 920, the angle between the movement direction of the force-applying block 920 and the second edge surface is 105 degrees, and the angle between the movement direction of the force-applying block 920 and the first edge surface is 75 degrees. In the circumferential direction of the instrument channel 9101, the angle between the movement directions of two adjacent force-applying blocks 920 is 30 degrees.

The force-applying blocks 920 have the same shape, which facilitates mass production; also, during the installation of the force-applying blocks 920, there is no need to identify the type of the force-applying blocks 920, which simplifies the assembly of the force-applying blocks 920.

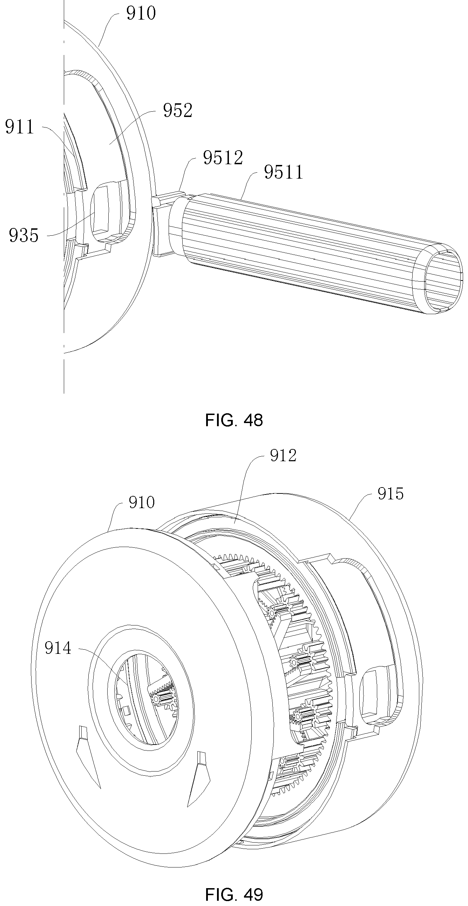

Referring to FIGS. 49 to 51, the detail of the housing 910 is shown. In one embodiment, the housing 910 is in the shape of a hollow disk, and the instrument channel 9101 extends through the axis of the disk. The interior of the housing 910 is the installation chamber 912, and the ring gear 930 and the force-applying blocks 920 are all located in the installation chamber 912. In one embodiment, the instrument channel 9101 is located at the center of the housing 910.

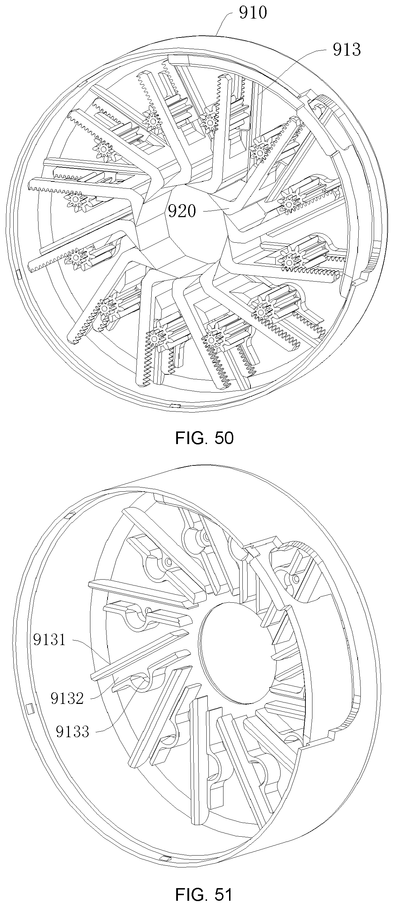

In order to limit the movement paths of the force-applying blocks 920, with reference to FIGS. 50 to 51, the present disclosure discloses guide units 913. The guide unit 913 is used to limit the movement direction of the force-applying block 920. The guide unit 913 is a guide rail arranged along a predetermined direction. There are no strict limitations on the shape and construction of the guide rail. The guide rail can have a groove or ridge arranged along the predetermined direction on the surface, or be a guide rod, or be a structure having an outer surface arranged along the predetermined direction, such as a blocking plate arranged along the predetermined direction, etc. In order to adapt to the guide unit 913, the force-applying block 920 has a sliding seat corresponding to the shape of the guide unit 913. For example, in the case where the guide unit 913 is a guide rod, the corresponding sliding seat can be a sliding sleeve surrounding the guide rod; in the case where the guide unit is an elongated groove, the corresponding sliding seat can be a support block or support bar received in the elongated groove.

Referring to FIGS. 50 to 51, the present disclosure discloses guide units 913. The guide unit 913 includes a guide groove 9132 and a guide block. The guide groove 9132 is opened in one of the housing 910 and the force-applying block 920, and the guide block is provided on the other of the housing 910 and the force-applying block 920 and engaged with the guide groove 9132. The guide groove 9132 can limit the movement path of the force-applying block 920 so that the force-applying block 920 moves along the preset path. In this embodiment, the guide groove 9132 is opened on the inner wall of the housing 910, and the guide block is provided on the force-applying block 920. In order to make the movement of the force-applying block 920 more stable, in one embodiment, for the same force-applying block 920, two guide units 913 are provided which are located on two opposite sides of the force-applying block 920 in the extension direction of the instrument channel 9101.

Referring to FIGS. 50 to 51, in this embodiment, the guide groove 9132 is formed between two guide plates 9131 that are fixed on the inner wall of the housing 910. The guide plates 9131 and the housing 910 are formed in one piece to enhance the connection strength between the guide plates 9131 and the housing 910, and also to reduce the difficulty of the processing the guide plates 9131 and the housing 910.

In this embodiment, the two guide plates 9131 are arranged in parallel. In the circumferential direction of the instrument channel 9101, the two opposite sides of the guide block are respectively in contact with the inner walls of the guide groove 9132 to prevent the force-applying block 920 from shaking in the circumferential direction of the instrument channel 9101.

In this embodiment, the guide block and the force-applying block 920 are formed in one piece. The guide block is an integral part of the force-applying block 920, which can strengthen the connection strength between the guide plates 9131 and the housing 910, and can also reduce the difficulty of the processing the guide plates 9131 and the housing 910. Alternatively, in other embodiments, the guide block and the force-applying block 920 can be formed in separate pieces, and the guide block is fixed on the housing 910 by welding or bonding.

When the guide block is installed in the guide groove 9132, in order to reduce the installation difficulty, in one embodiment, the opening of the guide groove 9132 faces the force-applying block 920, and the opening is flared so that it can guide the guide block to enter the guide groove 9132.

In this embodiment, at least part of the second gear 9422 is located in the guide groove 9132. In the case where the addendum circle of the second gear 9422 is larger than the width of the guide groove 9132, the guide groove 9132 is locally enlarged to form an averted area 9133 for accommodating the second gear 9422. In the case where the addendum circle of the second gear 9422 is smaller than the width of the guide groove 9132, part of the second gear 9422 can be directly received in the guide groove 9132.

In this embodiment, the crimper 9100 further includes transmission mechanisms 940, which are located in the installation chamber 912 and connected between the ring gear 930 and the force-applying blocks 920 in a transmission way. In this embodiment, the crimper 9100 further includes a driving mechanism 950 for driving the ring gear 930 to rotate. The driving mechanism 950 can be completely located in the installation chamber 912, or at least a part of the driving mechanism 950 is located outside the installation chamber 912.

In this embodiment, the housing 910 includes separate pieces that are snapped into each other. The separate pieces that are snapped into each other include at least two pieces, and the pieces are arranged in the axial direction of the housing 910. The snapped pieces include a main body 915 with an opening and a cover 916 snapped at the opening of the main body 915. The main body 915 has a chamber, the ring gear 930, the force-applying blocks 920 and the transmission mechanisms 940 are all located in the chamber. The instrument channel 9101 extends through the cover 916 and the main body 915 in sequence.

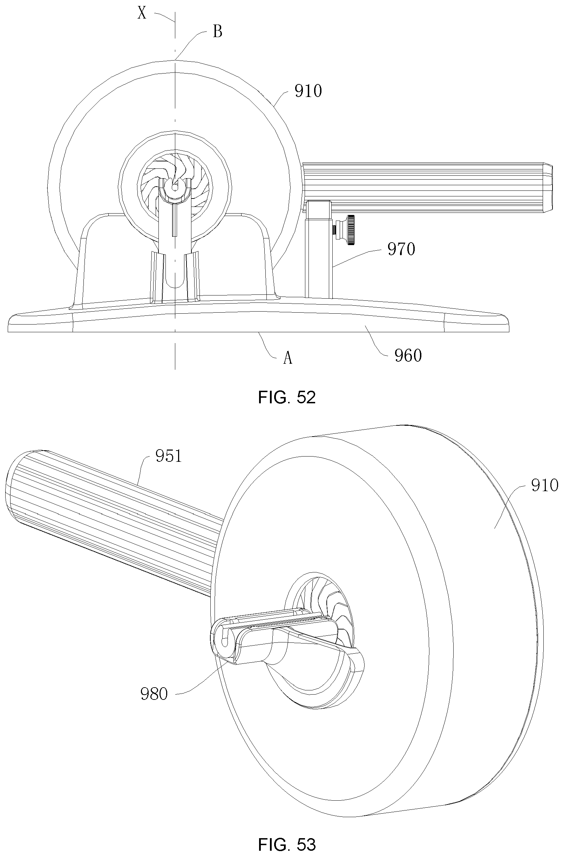

Referring to FIG. 40 and FIG. 52, in this embodiment, the crimper 9100 further includes a base 960, and the housing 910 is detachably installed on the base 960. The crimper 9100 is fixed on the support table through the base 960. The crimper 9100 has a bottom surface (e.g., depicted as A in FIG. 52) that matches the support table, and a top surface (e.g., depicted as B in FIG. 52) that is opposite to the bottom surface. Unless otherwise defined, the bottom surface of the crimper 9100 should be understood as the bottom surface of the base 960. Since the crimper 9100 can be set at a variety of angles, at different setting angles, the vertically downward side may be the side A or not, so the bottom side here does not refer to the downward side in use.

Regarding the fixation of the housing 910 on the base 960, referring to FIG. 40, in this embodiment, the crimper 9100 further includes a snap-fit connection structure 961. The housing 910 can be detachably installed on the base 960 through the snap-fit connection structure 961. The snap-fit connection structure 961 can fix the housing 910 to the base 960 and also facilitate the detachment of the housing 910 from the base 960. Depending on the installation position of the housing 910 and the base 960, various snap-fit connections known in the art can be used, such as nuts and bolts, bonding, etc.

Referring to FIG. 40, the present disclosure discloses a snap-fit connection structure 961, including a snapping tongue 9611 and a snapping slot 9612 that fits with the snapping tongue 9611. The snapping tongue 9611 is provided on one of the base 960 and the housing 910, and the snapping slot 9612 is opened in the other of the base 960 and the housing 910. When the housing 910 is installed on the base 960 at the predetermined position, the snapping tongue 9611 and the snapping slot 9612 are in snap-fit connection and fixed to each other so as to fix the housing 910 to the base 960. When detaching the housing 910 from the base 960, it is only necessary to detach the snapping tongue 9611 from the snapping slot 9612 and thus detach the housing 910 from the base 960.

Referring to FIG. 40, in this embodiment, the base 960 has an installation groove 962 that fits with the housing 910, and the snap-fit connection 961 is provided between the groove wall of the installation groove 962 and the housing 910. The installation groove 962 is opened away from the bottom of the base 960, and the housing 910 enters through the opening of the installation groove 962 until the snapping tongue 9611 and the snapping slot 9612 are in snap-fit connection and fixed to each other, where part of the housing 910 is received in the installation groove 962.

After the housing 910 is received in the installation groove 962, in the extension direction of the instrument channel 9101, the two axial ends of the housing 910 fit with the two opposite sides of the installation groove 962 respectively. The bottom wall of the installation groove 962 fits with the outer periphery of the housing 910 (in this embodiment, the bottom wall of the installation groove 962 is in an arc shape matching the housing 910).

The snapping tongue 9611 and the inner wall of the installation groove 962 are formed in one piece to enhance the connection strength between the snapping tongue 9611 and the housing 910 and reduce the processing difficulty of the snapping tongue 9611 and the housing 910. In order to facilitate the engagement between the snapping tongue 9611 and the snapping slot 9612, the snapping tongue 9611 and/or the snapping slot 9612 have a guide wedge, and the guide wedge can guide the snapping tongue 9611 to enter the snapping slot 9612.

In order to stably fix the housing 910 on the mounting base, in one embodiment, there are at least two groups of snap-fit connection structures 961 which are respectively located on two opposite axial sides of the housing 910. In other embodiments, on the same side of the housing 910, multiple groups of the snap-fit connection structures 961 are provided.

In this embodiment, in use of the crimper 9100, the lowest point and the highest point of the stroke of the driving handle 951 are located on the same side of the longitudinal cross-section of the housing 910 (e.g., depicted as X in FIG. 52), and the longitudinal cross-section is perpendicular to the bottom surface of the base 960 and passes through the axis of housing 910. In one embodiment, in use of the crimper 9100, the central angle between the highest point of the stroke of the driving handle and the highest point of the housing 910 is 40 degrees to 60 degrees. Preferably, the central angle between the highest point of the stroke of the driving handle and the highest point of the housing 910 is 40 degrees.

Referring to FIG. 40, in this embodiment, the crimper 9100 further includes a limiting component 970. The limiting component 970 directly or indirectly interferes with at least one of the following components: the ring gear 930; the force-applying blocks 920; the transmission mechanisms 940; and the driving mechanism 950.

The limiting component 970 is mainly used to limit the gathered state of the force-applying blocks 920, and there are no strict limitations on the shape and specific structure of the limiting component 970. For example, a lock pin, a frame structure or a solid component can be used. The general requirement is that the limiting component 970 should at least have sufficient mechanical strength and have a firm connection with the above components. Regarding the specific construction of the limiting component 970, please refer to the detailed description of the limiting component 970 below, which will not be elaborated here.

The crimped interventional instrument 990 needs to be loaded into the sheath. In order to support the sheath, with reference to FIG. 40, the present disclosure discloses a support 980. The support 980 is used to support the interventional instrument 990 or the delivery system. The support 980 is installed on the housing 910 or the base 960, and has a support position corresponding to the position of the instrument channel 9101. When the sheath is placed at the support position, the channel in the sheath faces the instrument channel, so that the interventional instrument 990 can enter the channel in the sheath. Regarding the specific construction of the support 980, please refer to the detailed description of the support 980 below, which will not be elaborated here.

Referring to FIGS. 36 to 40, the present disclosure further provides a crimper 9100, which includes:
a housing 910 which has a through instrument channel 9101;
a plurality of force-applying blocks 920, which are movably installed in the housing 910 and distributed around the instrument channel 9101. The plurality of force-applying blocks 920 have a gathered state (for example, the state of the force-applying blocks 920 shown in FIGS. 38) and a relative separated state (for example, the state of the force-applying blocks 920 shown in FIG. 39), and can contract and expand the instrument channel 9101 during switching the states;
a driving handle 951 that is linked with the force-applying blocks 920 to synchronously drive the force-applying blocks 920 to switch the states; and
a limiting component 970 which includes a fixed part 971 connected to the housing 910 and an adjustable part 972 that movably cooperates with the fixed part 971. The adjustable part 972 is configured to abut and limit the driving handle 951 that moves to the utmost position, where the force-applying blocks 920 are in the gathered state.

Before the interventional instrument 990 enters the instrument channel 9101, the plurality of force-applying blocks 920 are in the separated state. After the interventional instrument 990 enters the instrument channel 9101, the driving handle 951 drives the plurality of force-applying blocks 920 to gradually switch from the separated state to the gathered state. During the state switching process of the plurality of force-applying blocks 920, the plurality of force-applying blocks 920 will contract the instrument channel 9101, and the wall of the instrument channel 9101 will press the interventional instrument 990. The wall of the instrument channel 9101 will uniformly reduce the size of the interventional instrument 990 until the driving handle 951 abuts the adjustable part 972, thereby crimping the interventional instrument 990 by the crimper 9100. If adjustment on the gathered state of the force-applying blocks 920 is desired, the stroke of the driving handle 951 will change, and accordingly the position relationship between the adjustable part 972 and the fixed part 971 should be adjusted.

Referring to FIG. 40 and FIG. 52, in this embodiment, the crimper 9100 further includes a base 960, and the housing 910 is detachably installed on the base 960. The fixed part 971 of the limiting component 970 is installed to the base 960. The crimper 9100 is fixed on the support table through the base 960. The crimper 9100 has a bottom surface (e.g., depicted as A in FIG. 52) that matches the support table, and a top surface (e.g., depicted as B in FIG. 52) that is opposite to the bottom surface.

If the adjustment path of the limiting component 970 is not perpendicular to the bottom surface of the base 960, the force from the driving handle 951 acting on the limiting component 970 will focus on the connection between the limiting component 970 and the base 960, thereby increasing the force at the connection between the limiting component 970 and the base 960. In order to avoid such situation, in one embodiment, in use, the bottom surface of the base 960 is the support surface, and the adjustment path of the limiting component 970 is perpendicular to the support surface.

The limiting component 970 is made from a rod. Alternatively, in order to reduce the weight, a tube can be used. No matter a tube or a solid rod is used, they are both provided for supporting purpose. Therefore, the rod here is not strictly limited to be solid or hollow, which can be straight or partially curved along its spatial direction. Similarly, the cross-sectional shape is not strictly limited.

In this embodiment, the limiting component 970 has a length direction, and the length of the limiting component 970 in its own length direction is adjustable, so that the limiting component 970 can be quickly adjusted to the desired position.

In order to make the structure of the limiting component 970 compact, in this embodiment, the fixed part 971 and the adjustable part 972 are slidably matched with each other with one inserted in the other. The fixed part 971 can be inserted into the interior of the adjustable part 972, or the adjustable part 972 can be inserted into the interior of the fixed part 971, in order to reduce the length of the limiting component 970 in the radial direction.

In one embodiment, one of the fixed part 971 and the adjustable part 972 is a tubular structure, and the other is disposed in the inner cavity of the tubular structure. The tubular structure has its own inner cavity. The tubular structure not only has a certain support strength, but also can reduce the weight.

In order to prevent the adjustable part 972 from rotating in the inner cavity of the tubular structure, the fixed part 971 is provided with a limiting groove in its own axis, and the adjustable part 972 is locally recessed to form a limiting portion that fits with the limiting groove; and/or
both the inner cavity of the fixed part 971 and the radial cross-section of the adjustable part 972 are non-circular.

In this embodiment, the limiting component 970 further includes a locking mechanism 973 for maintaining the relative position between the fixed part 971 and the adjustable part 972. The locking mechanism 973 is used to fix the adjustable part 972 to the fixed part 971, and also allows the adjustable part 972 to move along the fixed part 971. Various locking methods known in the prior art can be used.

Regarding the specific construction of the locking mechanism 973, referring to FIG. 40, in this embodiment, the locking mechanism 973 includes a screw joint. The screw joint is provided on one of the fixed part 971 and the adjustable part 972, and can abut against the other to maintain the relative position between the fixed part 971 and the adjustable part 972.

The screw joint includes a stud 9731 and an operation portion 9732 fixed at one end of the stud 9731. The fixed part 971 is provided with a threaded hole 9733 that communicates with the interior of the fixed part 971. The stud 9731 is screwed in the threaded hole 9733, and can be operated through the operation portion 9732. After the adjustable part 972 moves to the predetermined position, the operator holds and rotates the operation portion 9732 to screw the stud 9731 into the threaded hole 9733 until the end of the stud 9731 facing away from the operation portion 9732 abuts against the adjustable part 972.

In this embodiment, one end of the adjustable part 972 has a support groove 9721, and is configured to abut and limit the driving handle 951 through the support groove 9721. The support groove 9721 can increase the contact area between the adjustable part 972 and the driving handle 951. The support groove 9721 is generally U-shaped, and the opening of the U-shape faces the driving handle 951.

The mechanism for driving the force-applying blocks 920 can refer to the previous embodiments. For example, the driving handle 951 is linked with the force-applying blocks 920 through the following components:
a ring gear 930 which is rotatably engaged with the housing 910, and can synchronously drive the plurality of force-applying blocks 920 to switch the states, the driving handle 951 is indirectly or directly connected to the ring gear 930;
racks 941 fixed to the corresponding force-applying blocks 920; and
transmission gears 942 rotatably installed in the housing 910. Each rack 941 meshes with the ring gear 930 through one or more transmission gears 942.

The ring gear 930 synchronously drives the plurality of force-applying blocks 920 based on meshing transmission to switch between the gathered state and the separated state. The meshing transmission can improve the stability and accuracy of the ring gear 930 driving the plurality of force-applying blocks 920. During the rotation of the ring gear 930, the racks 941 will be synchronously driven by the transmission gears 942 to move so as to drive the force-applying blocks 920 to move. Here, the ring gear 930, the transmission gears 942 and the racks 941 mesh with each other, so that the ring gear 930 can stably drive the force-applying blocks 920 to move.

In this embodiment, multiple groups of transmission gears 942 are provided, and each group corresponds to a force-applying block 920. The transmission gears 942 have the same shape, which facilitates mass production; also, during the installation of the transmission gears 942, there is no need to identify the type of the transmission gears 942, which simplifies the assembly of the transmission gears 942.

Referring to FIGS. 36 to 40, the present disclosure further provides a crimper 9100, which includes:
a housing 910 which has a through instrument channel 9101;
a plurality of force-applying blocks 920, which are movably installed in the housing 910 and distributed around the instrument channel 9101. The plurality of force-applying blocks 920 have a gathered state and a relative separated state, and can contract and expand the instrument channel 9101 during switching the states; and
a support 980 which includes a bear portion 983 and a support portion 984 that are connected to each other. The bear portion 983 matches the housing 910, and the support portion 984 has a support position corresponding to the position of the instrument channel 9101.

Before the interventional instrument 990 enters the instrument channel 9101, the plurality of force-applying blocks 920 are in the separated state. After the interventional instrument 990 enters the instrument channel 9101, the plurality of force-applying blocks 920 are driven to gradually switch from the separated state to the gathered state. During the state switching process of the plurality of force-applying blocks 920, the plurality of force-applying blocks 920 will contract the instrument channel 9101, and thus the wall of the instrument channel 9101 will press the interventional instrument 990. The wall of the instrument channel 9101 will uniformly reduce the size of the interventional instrument 990 until completing the crimping of the interventional instrument 990 by the crimper 9100.

After the crimper 9100 crimps the interventional instrument 990, the plurality of force-applying blocks 920 are driven to switch from the gathered state to the separated state, and then the interventional instrument 990 is moved into the sheath located on the support 980. When the sheath is placed at the support position, the channel in the sheath faces the instrument channel, so that the interventional instrument 990 can enter the channel in the sheath.

Referring to FIG. 40 and FIG. 52, in this embodiment, the crimper 9100 further includes a base 960, and the housing 910 is detachably installed on the base 960. The bear portion 983 can be inserted into the base 960. The crimper 9100 is fixed on the support table through the base 960. The crimper 9100 has a bottom surface (e.g., depicted as A in FIG. 52) that matches the support table, and a top surface (e.g., depicted as B in FIG. 52) that is opposite to the bottom surface.

In this embodiment, a slot extending in a straight line is provided on the outer wall of the housing 910, and the bear portion 983 is slidably fitted in the slot, so that the movement direction of the bear portion 983 after being inserted into the slot is linear. In one embodiment, one end of the slot serves as the entrance and is adjacent to the instrument channel 9101, and the other end of the slot extends toward the base 960.

In this embodiment, the extension direction of the slot is consistent with the radial direction of the instrument channel, and the entrance of the slot is aligned with the instrument channel 9101. In use of the crimper 9100, the slot is located below the instrument channel 9101.

In this embodiment, the bear portion 983 and the support portion 984 are formed in one piece to enhance the structural strength of the bear portion 983 and the support portion 984 and reduce the processing difficulty of the support 980. Alternatively, in other embodiments, the bear portion 983 and the support portion 984 can be formed in separate pieces, and the bear portion 983 and the support portion 984 can be fixed by a bolt or welding.

In this embodiment, the support position extends in the direction of the instrument channel 9101, where a support channel 981 is formed that is docked to the instrument channel 9101. In order to facilitate the observation of the sheath at the support position and to adjust the sheath in time, in one embodiment, the top of the support 980 is a semi-cylinder with a U-shaped cross section, and the top surface of the semi-cylinder is opened, with the interior of the semi-cylinder as the support channel 981.

In order to protect the outside of the sheath that would be further clamped, referring to FIG. 40, in this embodiment, an elastic liner 982 is provided in the support channel 981, and the elastic liner 982 is installed on the inner wall of the support channel 981. The elastic liner 982 is made of silicone. Alternatively, in other embodiments, the inner wall of the support channel 981 can be made of silicone, and in this case, the elastic liner 982 can be removed.

Regarding the fixation of the elastic liner and the support channel, referring to FIG. 40, in this embodiment, the elastic liner 982 is installed in the support channel 981 by snapping, bonding or fastener, so that the elastic liner 982 can be easily assembled to or disassembled from the support channel 981.

In this embodiment, one of the inner wall of the support channel 981 and the elastic liner 982 is provided with a groove 9821, and the other is provided with a snapping block 9811 that fits with the groove 9821. In the extension direction of the support channel, there are a plurality of snapping blocks 9811 and grooves 9821, and each snapping block 9811 is respectively snapped into the corresponding groove 9821.

Referring to FIG. 40, in this embodiment, the bear portion 983 is plate-shaped and is attached to the outer wall of the housing 910. Referring to FIG. 40, in this embodiment, the bear portion 983 has a length direction, one end of the bear portion 983 in the length direction fits with the slot, and the other end is connected with the support portion 984.

In order to enhance the connection strength between the semi-cylinder and the bear portion, in this embodiment, a reinforcing rib is fixed between the bottom of the semi-cylinder and the bear portion. The reinforcing rib is plate-shaped, and the extension direction of the semi-cylinder is perpendicular to the extension direction of the bear portion. When the reinforcing rib is located at the connection between the semi-cylinder and the bear portion, the two sides of the reinforcing rib are connected with the semi-cylinder and the bear portion.

The mechanism for driving the force-applying blocks 920 can refer to the previous embodiments. For example, the crimper 9100 further includes:
a ring gear 930 which is rotatably engaged with the housing 910, and can synchronously drive the plurality of force-applying blocks 920 to switch the states;
racks 941 fixed to the corresponding force-applying blocks 920; and
transmission gears 942 rotatably installed in the housing 910. Each rack 941 meshes with the ring gear 930 through one or more transmission gears 942.

The ring gear 930 synchronously drives the plurality of force-applying blocks 920 based on meshing transmission to switch between the gathered state and the separated state. The meshing transmission can improve the stability and accuracy of the ring gear 930 driving the plurality of force-applying blocks 920. During the rotation of the ring gear 930, the racks 941 will be synchronously driven by the transmission gears 942 to move so as to drive the force-applying blocks 920 to move. Here, the ring gear 930, the transmission gears 942 and the racks 941 mesh with each other, so that the ring gear 930 can stably drive the force-applying blocks 920 to move.

In this embodiment, multiple groups of transmission gears 942 are provided, and each group corresponds to a force-applying block 920. The transmission gears 942 have the same shape, which facilitates mass production; also, during the installation of the transmission gears 942, there is no need to identify the type of the transmission gears 942, which simplifies the assembly of the transmission gears 942.

Referring to FIGS. 36 to 40 and FIGS. 53 to 54, the present disclosure further provides a crimper 9100, including:
a housing 910 which has a through instrument channel 9101;
a plurality of force-applying blocks 920, which are movably installed in the housing 910 and distributed around the instrument channel 9101. The plurality of force-applying blocks 920 have a gathered state and a relative separated state, and can contract and expand the instrument channel 9101 during switching the states; and
a support 980 which is detachably mounted on the housing 910 by magnetic attraction, and has a support position corresponding to the position of the instrument channel 9101.

In this embodiment, one of the support 980 and the housing 910 is provided with a magnetic piece 985, and the other is provided with a matching piece 986 that magnetically engages with the magnetic piece 985. One end of the support 980 facing the housing 910 is attached to the housing 910, and the magnetic piece 985 is provided at the contacting surface of the support 980 attaching to the housing 910. In this embodiment, the matching piece 986 is made of ferromagnetic material.

In order to fix the support 980 on the housing 910 more firmly, in this embodiment, a plurality of magnetic pieces 985 are provided, and at least two magnetic pieces 985 are offset from each other in the circumferential direction of the instrument channel. Furthermore, in this embodiment, the magnetic pieces 985 are arranged sequentially in the circumferential direction of the instrument channel.

In this embodiment, the end surfaces of the support 980 and the housing 910 that are in contact with each other each have an installation position, and the magnetic piece 985 and the matching piece 986 are fixed at the corresponding installation positions. In order to reduce the gap between the support 980 and the housing 910, in one embodiment, the installation position is configured as a groove, and the magnetic piece 985 or the matching piece 986 is embedded in the corresponding groove, so that the end surface of the support 980 is in contact with the outer surface of the housing 910. In this embodiment, the support position extends in the direction of the instrument channel 9101 and forms a support channel 981 that is docked to the instrument channel 9101.

One of the magnetic piece 985 and the matching piece 986 protrudes from the corresponding installation position, and the other is embedded in the bottom of the groove. The piece that protrudes from the installation position can extend into the corresponding groove, and attract the piece located in the groove. In this embodiment, the magnetic piece 985 is installed on the support 980, and the matching piece 986 is installed on the housing 910. The magnetic piece 985 protrudes from the end face of the support 980, and the matching piece 986 is embedded in the bottom of the groove. When installing the support 980 on the housing 910, the magnetic piece 985 on the support 980 extends into the groove of the housing 910 and magnetically attracts the matching piece 986 in the groove so as to fix the support 980 on the housing 910.

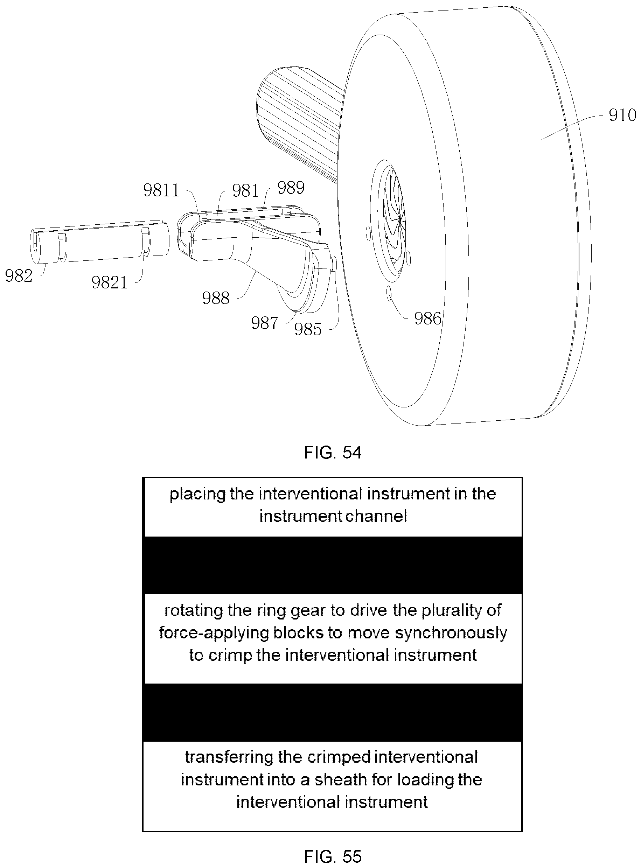

Regarding the specific construction of the support 980, in one embodiment and referring to FIG. 54, the support 980 includes:
a semi-cylinder 989 with a U-shaped cross section, the top surface of the semi-cylinder 989 is open, and the interior thereof serves as the support channel 981;
an attachment portion 987 magnetically attached and fixed to the housing 910, the attachment portion 987 is located at the periphery of the instrument channel 9101 and extends in the circumferential direction of the instrument channel 9101; and
a flared section 988 fixedly connected between the semi-cylinder 989 and the attachment portion 987. In the axial direction of the instrument channel, the small mouth of the flared section 988 is connected to the middle of the semi-cylinder 989, and the large mouth is connected to the attachment portion 987.

The semi-cylinder 989 has a U-shaped cross section in the radial direction of the instrument channel. In use of the crimper 9100, the U-shaped opening faces upward so that the top surface of the semi-cylinder 989 is open. The installation position is on the side of the attachment portion 987 facing the housing 910. In the radial direction of the instrument channel, the size of the attachment portion 987 is larger than the wall thickness of the semi-cylinder 989 and the wall thickness of the flared section 988 to increase the contact area between the support 980 and the housing 910. In one embodiment, the corresponding central angle of the attachment portion 987 is 120 degrees to 180 degrees, and the attachment portion 987 is located below the instrument channel 9101. The attachment portion 987 is plate-shaped, and the attachment portion 987 and the semi-cylinder 989 are formed in one piece.

The flared section 988 is in the shape of a semi-cylinder, and the flared section 988 has a U-shaped cross section in the radial direction of the instrument channel. In use of the crimper 9100, the top of the flared section 988 is open. In the extension direction of the instrument channel, the channel in the flared section 988 gradually increases from the middle of the semi-cylinder 989 toward the housing 910 to form a large mouth and a small mouth at two ends of the flared section 988.

The small mouth of the flared section 988 is arranged circumferentially around the semi-cylinder 989 in use and is connected to the outer wall of the semi-cylinder. There is a certain gap between the large mouth of the flared section 988 and the semi-cylinder 989. The top surface of the semi-cylinder 989 is substantially flush with the top surface of the flared section 988, so that part of the semi-cylinder 989 is located within the flared section 988. In one embodiment, in the radial direction of the instrument channel, the semi-cylinder 989 is located in the middle of the instrument channel 9101, and there is a gap between it and the attachment portion 987.

In order to protect the outside of the sheath that would be further clamped, referring to FIG. 54, in this embodiment, an elastic liner 982 is provided in the support channel 981, and the elastic liner 982 is installed on the inner wall of the support channel 981. The elastic liner 982 is made of silicone. Alternatively, in other embodiments, the inner wall of the support channel 981 can be made of silicone, and in this case, the elastic liner 982 can be removed.

Regarding the fixation of the elastic liner and the support channel, referring to FIG. 54, in this embodiment, the elastic liner 982 is installed in the support channel 981 by snapping, bonding or fastener, so that the elastic liner 982 can be easily assembled to or disassembled from the support channel 981.

In this embodiment, one of the inner wall of the support channel 981 and the elastic liner 982 is provided with a groove 9821, and the other is provided with a snapping block 9811 that fits with the groove 9821. In the extension direction of the support channel, there are a plurality of snapping blocks 9811 and grooves 9821, and each snapping block 9811 is respectively snapped into the corresponding groove 9821.

The mechanism for driving the force-applying blocks 920 can refer to the previous embodiments. For example, the crimper 9100 further includes:
a ring gear 930 which is rotatably engaged with the housing 910, and can synchronously drive the plurality of force-applying blocks 920 to switch the states;
racks 941 fixed to the corresponding force-applying blocks 920; and
transmission gears 942 rotatably installed in the housing 910. Each rack 941 meshes with the ring gear 930 through one or more transmission gears 942.

The ring gear 930 synchronously drives the plurality of force-applying blocks 920 based on meshing transmission to switch between the gathered state and the separated state. The meshing transmission can improve the stability and accuracy of the ring gear 930 driving the plurality of force-applying blocks 920. During the rotation of the ring gear 930, the racks 941 will be synchronously driven by the transmission gears 942 to move so as to drive the force-applying blocks 920 to move. Here, the ring gear 930, the transmission gears 942 and the racks 941 mesh with each other, so that the ring gear 930 can stably drive the force-applying blocks 920 to move.

In this embodiment, multiple groups of transmission gears 942 are provided, and each group corresponds to a force-applying block 920. The transmission gears 942 have the same shape, which facilitates mass production; also, during the installation of the transmission gears 942, there is no need to identify the type of the transmission gears 942, which simplifies the assembly of the transmission gears 942.

Referring to FIGS. 55 to 58, an embodiment of the present disclosure further provides a method for loading an interventional instrument 990, including:
providing a crimper 9100 according to the above embodiment;
placing the interventional instrument 990 in the instrument channel 9101;
rotating the ring gear 930 to drive the plurality of force-applying blocks 920 to move synchronously to crimp the interventional instrument 990; and
transferring the crimped interventional instrument 990 into a sheath for loading the interventional instrument 990.

Before loading the interventional instrument 990, the sheath is preinstalled on the support 980, and the force-applying blocks 920 are in the separated state. After placing the interventional instrument 990 in the instrument channel 9101, the driving handle 951 is pulled to drive the ring gear 930 to rotate and thus to drive the plurality of force-applying blocks 920 to switch to the gathered state, during which, the plurality of force-applying blocks 920 move synchronously to crimp the interventional instrument 990 until the interventional instrument 990 is crimped to the desired size. After the interventional instrument 990 is crimped to the desired size, the interventional instrument 990 is pushed so that the crimped interventional instrument 990 is transferred into the sheath. In order to facilitate loading of the interventional instrument 990 into the sheath, in one embodiment, the sheath is pre-positioned and aligned with the instrument channel 9101. The channel of the sheath is aligned with the instrument channel 9101.

Referring to FIG. 56, the specific shape of the interventional instrument 990 is not strictly limited. For example, it can include a stent 991 with a connecting ear 992 at one axial end of the stent 991. The connecting ear 992 can have an enlarged head at the end. The stent 991 has a radially compressible or expandable structure, generally a mesh-shaped cylinder formed by cutting or braiding. In this embodiment, the interventional instrument 990 is a heart valve prosthesis.

The interventional instrument 990 has an axial direction. In the case where the radial sizes of different sections of the interventional instrument 990 in its own axial direction are different, in one embodiment, depending on the different radial sizes of the interventional instrument 990 at different sections, the interventional instrument 990 is crimped stepwise. The interventional instrument 990 includes a plurality of sections depending on the different radial sizes, and the sections of the interventional instrument 990 are crimped respectively by the crimper 9100 until the interventional instrument 990 can be loaded into the sheath.

Referring to FIGS. 56 to 57, in this embodiment, the interventional instrument 990 includes a first section 993 and a second section 994 in its axial direction. The radial size of the first section 993 is larger than that of the second section 994. The stepwise crimping method includes: inserting a mandrel inside the interventional instrument 990, and crimping the first section 993 so that the radial size of the first section 993 is approximately equal to the radial size of the second section 994, wherein the axis of the first section 993 is generally consistent with the axis of the second section 994.

Before crimping the interventional instrument 990, the dry interventional instrument 990 needs to be moistened. The second section 994 of the interventional instrument 990 is hold by a clamp, and the first section 993 is placed in the instrument channel 9101. The clamp abuts the housing 910 to limit the position of the first section 993 in the instrument channel 9101, the interventional instrument 990 is then adjusted so that the axis of the interventional instrument 990 is collinear with the axis of the instrument channel 9101, and finally the first section 993 is crimped. The specific shape of the clamp is not strictly limited. For example, the clamp can be cylindrical, and the second section 994 of the interventional instrument 990 can be placed inside the clamp.

The crimped interventional instrument 990 is removed from the clamp. In order to reduce the radial size of the interventional instrument 990, in one embodiment, the stepwise crimping method further includes: inserting a mandrel inside the interventional instrument, and then synchronously crimping the first section 993 and the second section 994 until in contact with the mandrels. Before the interventional instrument 990 is crimped, a protective cover is arranged around the outside of the interventional instrument 990. The protective cover can prevent the interventional instrument 990 from being damaged by the crimper 9100. The specific shape of the mandrel is not strictly limited, for example, the mandrel is cylindrical.

Referring to FIG. 58, in this embodiment, the crimped interventional instrument 990 is loaded into a delivery system 9103. The delivery system 9103 can be used to treat heart valves (e.g., mitral valve, aortic valve, tricuspid valve, vena cava valve, pulmonary valve). The treatment can include, but is not limited to, valve replacement surgery, valve repair, or other surgeries that affects valve function.

Referring to FIG. 58, in this embodiment, the delivery system 9103 includes two sheaths arranged coaxially from inside to outside, and a control handle that drives the two sheaths to move relative to each other. The distal ends of the sheaths are used to cooperate with each other to operate the interventional instrument 990, and the proximal ends of the sheaths are connected to the control handle which hydraulically drives the sheaths to move relative to each other.

The control handle here hydraulically drives the sheaths to operate the interventional instrument, such as releasing, cutting, rotating, grasping or recovering, etc. The entire hydraulic system is configured at the proximal end, which is more convenient for on-site commissioning or assembly. Even if an emergency occurs, the undesired situations can be easily avoided outside the human body. By contrast, if the hydraulic mechanism is configured at the distal end, it will impose more stringent requirements on the size and safety of the apparatus, and the possible motion mode and direction are also limited due to apparatus problems.

The two sheaths are a first sheath 9104 and a second sheath 9105 that are slidably arranged from inside to outside with one inserted in the other. The distal end of the first sheath 9104 is used to place the interventional instrument 990, and the second sheath 9105 is used to cover or release the interventional instrument 990. The most distal end of the first sheath 9104 is a guide head 9106, and an installation head 9107 is fixed adjacent to the proximal end of the guide head 9106. When the interventional instrument 990 is loaded, it is located between the guide head 9106 and the installation head 9107 and radially compressed. The interventional instrument 990 generally has a connecting ear 992 connected to the installation head 9107. When loading, the connecting ear 992 cooperates with the installation head 9107 to limit the axial position of the interventional instrument 990.

In this embodiment, the first sheath 9104 of the delivery system 9103 is inserted into the interventional instrument 990, the crimped interventional instrument 990 is in contact with the first sheath 9104, and the crimped interventional instrument 990 is pushed into the second sheath 9105 of the delivery system 9103. After assembling the interventional instrument 990 on the first sheath 9104, placing the second sheath 9105 on the support 980, and crimping the interventional instrument 990 to the predetermined size, the control handle drives the first sheath 9104 to slide back axially relative to the second sheath 9105, so that the interventional instrument 990 is received in the second sheath 9105. When crimping the interventional instrument 990, the operator can observe the interventional instrument 990 after a period of compression (for example, 10 seconds).

The first sheath member 9104 and the second sheath member 9105 are plastic sheaths or metal sheaths commonly used in the field of interventional instrument 990, such as cutting hypotubes or metal braided tubes or combinations of metal braided tube and hypotube. The first sheath 9104 and/or the second sheath 9105 can be multi-layer composite sheaths.

The technical features of the above embodiments can be arbitrarily combined, and not all possible combinations of the technical features of the above embodiments have been described for the sake of brevity of description. However, as long as there is no contradiction in the combination of these technical characteristics, such combination should be regarded as falling into the scope of this specification. When the technical features in different embodiments are shown in the same drawing, it can be considered that the drawing also discloses a combined embodiment of various embodiments involved.

The above-described embodiments only illustrate several embodiments of the present disclosure, and the description thereof is specific and detail, but should not be construed as limiting the scope of the patent disclosure. It should be noted that, for those of ordinary skill in the art, several modifications and improvements can be made without departing from the concept of the present disclosure, all of which fall into the protection scope of the present disclosure.

## Claims

1. A crimper for crimping an interventional instrument, comprising:
a housing with a through instrument channel;
a plurality of force-applying blocks which are movably installed in the housing and distributed around the instrument channel, the plurality of force-applying blocks have relative gathered and separated states and are configured to contract and expand the instrument channel during switching the states; and
a ring gear which is rotatably matched with the housing and configured to synchronously drive the plurality of force-applying blocks to switch the states.

2. The crimper for crimping the interventional instrument according to claim 1, wherein the crimper further comprises transmission mechanisms, and each transmission mechanism is connected between the ring gear and a corresponding force-applying block in a transmission manner.

3. The crimper for crimping the interventional instrument according to claim 2, wherein each transmission mechanism comprises:
a rack(s) provided on the corresponding force-applying block; and
a transmission gear(s) rotatably installed in the housing, wherein each rack meshes with the ring gear through one or more transmission gears.

4. The crimper for crimping the interventional instrument according to claim 1, wherein a rotation axis of the ring gear and an extension direction of the instrument channel are parallel to each other.

5. The crimper for crimping the interventional instrument according to claim 3, wherein a rotation axis of the transmission gear and the rotation axis of the ring gear are parallel to each other.

6. The crimper for crimping the interventional instrument according to claim 1, wherein the ring gear comprises an annular portion and driving teeth distributed on the annular portion in a transmission fit with the force-applying blocks, and the driving teeth are configured as at least one of the followings:
inner teeth distributed on an inner periphery of the annular portion;
outer teeth distributed on an outer periphery of the annular portion; and
side teeth distributed on an axial end surface of the annular portion.

7. The crimper for crimping the interventional instrument according to claim 6, wherein the number of the driving teeth is an integer multiple of the number of the force-applying blocks.

8. The crimper for crimping the interventional instrument according to claim 7, wherein the number of the driving teeth is 8 times the number of the force-applying blocks.

9. The crimper for crimping the interventional instrument according to claim 1, wherein the ring gear comprises an annular portion, and the annular portion is provided with a plurality of weight-reducing grooves in a circumferential direction of the ring gear.

10. The crimper for crimping the interventional instrument according to claim 1, wherein the ring gear surrounds an outer periphery of the plurality of force-applying blocks.

11. The crimper for crimping the interventional instrument according to claim 3, wherein the rack and the ring gear are offset from each other in an extension direction of the instrument channel.

12. The crimper for crimping the interventional instrument according to claim 3, wherein two racks are fixed on each force-applying block and arranged in parallel, and the two racks are respectively located on two sides of the ring gear in an extension direction of the instrument channel.

13. The crimper for crimping the interventional instrument according to claim 3, wherein the transmission gear uses 1 to 3-stage gear transmission, and depending on transmission sequence, an initial-stage gear meshes with the ring gear, and a terminal-stage gear meshes with the rack.

14. The crimper for crimping the interventional instrument according to claim 3, wherein multiple groups of transmission gears are provided with each group corresponding to a force-applying block.

15. The crimper for crimping the interventional instrument according to claim 14, wherein each group of transmission gear comprises a first gear and a second gear coaxially fixed with the first gear, and the first gear meshes with the ring gear, and the second gear meshes with the rack on the corresponding force-applying block.

16. The crimper for crimping the interventional instrument according to claim 15, wherein for each force-applying block, each rack is configured with a second gear, or all racks are configured with a common second gear.

17. The crimper for crimping the interventional instrument according to claim 16, wherein a reference diameter of the first gear is D1, a reference diameter of the second gear is D2, and D1: D2 = 1: (0.3 to 0.7).

18. The crimper for crimping the interventional instrument according to claim 16, wherein an axial length of the first gear is L1, an axial length of the second gear is L2, and L1 : L2 = (3 to 6) : 1.

19. The crimper for crimping the interventional instrument according to claim 15, wherein a transmission ratio between the first gear and the ring gear is in a range of 1: (5 to 15); and
a transmission ratio between the second gear and the ring gear is in a range of 1 : (5 to 15).

20. The crimper for crimping the interventional instrument according to claim 19, wherein the transmission ratio between the first gear and the ring gear is 1: 9.6; and
the transmission ratio between the second gear and the ring gear is 1: 9.6.

21. The crimper for crimping the interventional instrument according to claim 19, wherein the transmission ratio between the first gear and the ring gear is 3: 26; and
the transmission ratio between the second gear and the ring gear is 3: 26.

22. The crimper for crimping the interventional instrument according to claim 3, wherein the rack(s) and the corresponding force-applying block are formed in one or separate pieces.

23. The crimper for crimping the interventional instrument according to claim 1, wherein the interventional instrument is a heart valve prosthesis.

24. The crimper for crimping the interventional instrument according to claim 1, wherein the crimper further comprises a driving mechanism for driving the ring gear to rotate, and the driving mechanism uses at least one of the following modes:
mode A: the driving mechanism is a driving handle indirectly or directly connected to the ring gear; and
mode B: the driving mechanism is an electrically-operated component connected to the ring gear in a transmission manner.

25. The crimper for crimping the interventional instrument according to claim 24, wherein the electrically-operated component is installed on the housing to drive the ring gear to rotate relative to the housing.

26. The crimper for crimping the interventional instrument according to claim 25, wherein the electrically-operated component is a motor.

27. The crimper for crimping the interventional instrument according to claim 24, wherein an operation window is opened in the housing, and at least a part of the ring gear is exposed to the operation window and connected to the driving handle.

28. The crimper for crimping the interventional instrument according to claim 27, wherein at least a part of the ring gear is a connection portion exposed to the operation window, and the driving handle is detachably fixed to the connection portion.

29. The crimper for crimping the interventional instrument according to claim 24, wherein a central angle corresponding to a stroke of the driving handle is in a range of 30 degrees to 120 degrees.

30. The crimper for crimping the interventional instrument according to claim 29, wherein the central angle corresponding to a stroke of the driving handle is in a range of 30 degrees to 60 degrees.

31. The crimper for crimping the interventional instrument according to claim 30, wherein the central angle corresponding to a stroke of the driving handle is 40 degrees.

32. The crimper for crimping the interventional instrument according to claim 24, wherein the driving handle comprises a holding portion and a snapping portion, and the ring gear is provided with a snapping slot that matches the snapping portion.

33. The crimper for crimping the interventional instrument according to claim 24, wherein the driving handle has a rod-shaped structure, one end of the driving handle is connected to the ring gear, and the other end extends away from the instrument channel.

34. The crimper for crimping the interventional instrument according to claim 1, wherein in process of the force-applying blocks switching from the gathered state to the separated state, two adjacent force-applying blocks are always against each other in a circumferential direction of the instrument channel.

35. The crimper for crimping the interventional instrument according to claim 1, wherein each force-applying block has a bent portion at one end toward the instrument channel;
in a circumferential direction of the instrument channel, the bent portion has a first edge surface on an outside and a second edge surface on an inside; and
a wall of the instrument channel is surrounded by intersections of the first edge surfaces and the second edge surfaces of the force-applying blocks.

36. The crimper for crimping the interventional instrument according to claim 35, wherein in the circumferential direction of the instrument channel, the first edge surface of one of two adjacent force-applying blocks is in contact with the second edge surface of the other force-applying block.

37. The crimper for crimping the interventional instrument according to claim 35, wherein in the circumferential direction of the instrument channel, the second edge surface of one of two adjacent force-applying blocks covers a part of the first edge surface of the other force-applying block, and the other part of the first edge surface exposed to the instrument channel is configured as an action surface; and
in process of the force-applying blocks switching from the gathered state to the separated state, a length of the action surface in the circumferential direction of the instrument channel gradually increases.

38. The crimper for crimping the interventional instrument according to claim 35, wherein the first edge surface and the second edge surface are each connected to an adjacent portion of a corresponding force-applying block through an arc-shaped transition surface.

39. The crimper for crimping the interventional instrument according to claim 35, wherein for each force-applying block, an angle between the first edge surface and the second edge surface in the circumferential direction of the instrument channel is 30 degree, an angle between a movement direction of the force-applying block and the second edge surface is 105 degrees, and an angle between the movement direction of the force-applying block and the first edge surface is 75 degrees.

40. The crimper for crimping the interventional instrument according to claim 1, wherein in a circumferential direction of the instrument channel, an angle between movement directions of two adjacent force-applying blocks is 30 degrees.

41. The crimper for crimping the interventional instrument according to claim 1, wherein the housing is in a shape of a hollow disc, the instrument channel extends through an axis of the disc, an interior of the housing is an installation chamber, and the ring gear and the force-applying blocks are all located in the installation chamber.

42. The crimper for crimping the interventional instrument according to claim 1, wherein the crimper further comprises an elastic piece installed on the housing; and
the ring gear is provided with a plurality of blocking teeth in its own circumferential direction for cooperating with the elastic piece, and the elastic piece is configured to move over the blocking teeth and vibrate or make sounds.

43. The crimper for crimping the interventional instrument according to claim 1, wherein at least one of the force-applying blocks is provided with an indicator, and the housing is provided with a view port corresponding to the indicator.

44. The crimper for crimping the interventional instrument according to claim 43, wherein the indicator and the corresponding force-applying block are formed in one or separate pieces, and the indicator is located on one side of the corresponding force-applying block in an axial direction of the housing.

45. The crimper for crimping the interventional instrument according to claim 44, wherein when the indicator and the corresponding force-applying block are formed in separate pieces, the indicator and the corresponding force-applying block are fixed together by insertion and/or bonding.

46. The crimper for crimping the interventional instrument according to claim 45, wherein one of the indicator and the corresponding force-applying block is provided with an insertion portion, and the other is provided with a slot that matches the insertion portion.

47. The crimper for crimping the interventional instrument according to claim 43, wherein the view port is elongated and extends in a movement direction of the corresponding force-applying block where the indicator is located.

48. The crimper for crimping the interventional instrument according to claim 43, wherein a movement direction of the corresponding force-applying block with the indicator is parallel to a bottom surface of the crimper.

49. The crimper for crimping the interventional instrument according to claim 43, wherein the view port is hollowed out of the housing, and a transparent cover is provided at the hollowed out portion.

50. The crimper for crimping the interventional instrument according to claim 49, wherein the transparent cover is snapped into the hollowed out portion of the housing.

51. The crimper for crimping the interventional instrument according to claim 43, wherein an end of the indicator extends out of the view port to form an indication portion, and a width of the indication portion is greater than a width of the view port.

52. The crimper for crimping the interventional instrument according to claim 51, wherein the housing has a recessed area surrounding the view port, and the indication portion is disposed in the recessed area; and
the view port is hollowed out of the housing, a transparent cover is provided at the hollowed out portion, and the transparent cover is snapped into the recessed area and is flush with a surrounding portion of the housing.

53. The crimper for crimping the interventional instrument according to claim 43, wherein there are at least two indicators, and the at least two indicators are located on two opposite sides of the housing in an axial direction of the housing.

54. The crimper for crimping the interventional instrument according to claim 53, wherein there are two indicators which are fixed on the same force-applying block.

55. The crimper for crimping the interventional instrument according to claim 51, wherein the indicator has a different color from the housing.

56. The crimper for crimping the interventional instrument according to claim 49, wherein the housing and/or the transparent cover are provided with a reference scale for showing displacement of the indicator.

57. A crimper for crimping an interventional instrument, comprising:
a housing which is hollow and has a through instrument channel;
a plurality of force-applying blocks which are movably installed in the housing and distributed around the instrument channel, the plurality of force-applying blocks can be relatively gathered and separated, thereby contracting and expanding the instrument channel;
a ring gear rotatably installed in the housing and located around the instrument channel, the ring gear is connected to the force-applying blocks in a transmission manner and configured to synchronously drive the plurality of force-applying blocks; and
a driving shaft, which has at least a part located in the housing and linked with the ring gear and at least a part for connecting a power source.

58. The crimper for crimping the interventional instrument according to claim 57, wherein a rotation axis of the driving shaft is parallel to a rotation axis of the ring gear.

59. The crimper for crimping the interventional instrument according to claim 57, wherein the power source is a knob and/or a motor;
at least part of the knob is exposed out of the housing, and the knob and the driving shaft are formed in one or separate pieces; and
the motor is located inside the housing.

60. The crimper for crimping the interventional instrument according to claim 59, wherein the crimper has opposite front and back surfaces in an extension direction of the instrument channel, the knob is located on the front surface of the housing, and a support is installed on the front surface of the housing.

61. The crimper for crimping the interventional instrument according to claim 59, wherein at least part of the knob is located outside the housing, and an elastic washer is pressed between said part and an outer wall of the housing.

62. The crimper for crimping the interventional instrument according to claim 61, wherein the elastic washer is annular and surrounds the knob, and wherein one axial side of the elastic washer is in contact with the housing, and the other side is in contact with the knob.

63. The crimper for crimping the interventional instrument according to claim 62, wherein the outer wall of the housing has a concave area corresponding to the knob, and the elastic washer is disposed in the concave area.

64. The crimper for crimping the interventional instrument according to claim 57, wherein the driving shaft is located outside the ring gear in a radial direction of the ring gear.

65. The crimper for crimping the interventional instrument according to claim 57, wherein the driving shaft is configured to directly drive the ring gear or through a linkage component; and
the linkage component uses a gear set or a worm gear for transmission.

66. The crimper for crimping the interventional instrument according to claim 65, wherein the gear set comprises a plurality of gears that mesh with each other for transmission, and a rotation axis of each gear is parallel to the driving shaft.

67. The crimper for crimping the interventional instrument according to claim 65, wherein the ring gear comprises an annular portion, and driving teeth are distributed on an outer periphery of the annular portion for meshing with the linkage component.

68. The crimper for crimping the interventional instrument according to claim 66, wherein the number of the gears is 2 to 5.

69. The crimper for crimping the interventional instrument according to claim 65, wherein the gear set comprises:
a third gear which is coaxially fixed with the driving shaft; and
a fourth gear rotatably matching with the housing, wherein the fourth gear is engaged between the third gear and the ring gear and used for transmission.

70. The crimper for crimping the interventional instrument according to claim 69, wherein the third gear and the fourth gear are both located in the housing, and in a radial direction of the ring gear, the third gear and the fourth gear are both located outside the ring gear.

71. The crimper for crimping the interventional instrument according to claim 69, wherein the ring gear comprises an annular portion, and driving teeth are distributed on an outer periphery of the annular portion for meshing with the linkage component;
the fourth gear comprises at least a first tooth unit and a second tooth unit arranged coaxially, meshing with the third gear and the driving teeth respectively; and
a tooth thickness ratio between the first tooth unit and the second tooth unit is in a range of 1:0.7 to 1.5.

72. The crimper for crimping the interventional instrument according to claim 71, wherein in an axial direction of the ring gear, an outer periphery of the ring gear is partially offset to form an offset area, and the driving teeth are located on the offset area; and
the ring gear has an averted area on a front side of the offset area for accommodating the first tooth unit or the second tooth unit.

73. A crimper for crimping an interventional instrument, comprising:
a housing which is hollow and has a through instrument channel;
a plurality of force-applying blocks which are movably installed in the housing and distributed around the instrument channel, the plurality of force-applying blocks can be relatively gathered and separated, thereby contracting and expanding the instrument channel;
a ring gear rotatably installed in the housing and located around the instrument channel, the ring gear is connected to the force-applying blocks in a transmission manner and configured to synchronously drive the plurality of force-applying blocks, and the ring gear is provided with a plurality of limiting structures located at different circumferential positions of the ring gear; and
a shifter movably installed in the housing and having a plurality of working positions, wherein the shifter is configured to cooperate with a corresponding limiting structure at each working position to limit rotation of the ring gear.

74. The crimper for crimping the interventional instrument according to claim 73, wherein the limiting structures have different radial positions on the ring gear, and the shifter is configured to move in a radial direction of the ring gear to corresponding working positions.

75. The crimper for crimping the interventional instrument according to claim 74, wherein each limiting structure is a limiting step provided on the ring gear, and the shifter is configured to abut against a corresponding limiting step in a circumferential direction of the ring gear at a corresponding working position.

76. The crimper for crimping the interventional instrument according to claim 73, wherein the crimper further comprises a driving mechanism for driving the shifter, and the driving mechanism comprises:
an operation button, which is rotatably mounted on the housing;
a transmission member, which is linked to the operation button and configured to drive the shifter to switch the working positions; and
an elastic member acting on the shifter to keep the shifter at a corresponding working position.

77. The crimper for crimping the interventional instrument according to claim 76, wherein the transmission member is a cam, and a rotation axis of the cam is perpendicular to a movement direction of the shifter.

78. The crimper for crimping the interventional instrument according to claim 76, wherein the operation button is located on a front surface of the housing, and the operation button and a view port on the same surface are respectively located on two sides of the instrument channel.

79. The crimper for crimping the interventional instrument according to claim 76, wherein the transmission member is a cam, and the shifter at least comprises a driving portion that abuts against an outer peripheral surface of the cam, a locking portion for cooperating with the corresponding limiting structure, and a transmission portion connected with the elastic member; and
in an axial direction of the ring gear, the driving portion and the locking portion are located on two opposite sides of the shifter.

80. The crimper for crimping the interventional instrument according to claim 79, wherein the elastic member is a compression spring, and the elastic member is pressed between the transmission portion and an inner wall of the housing for applying force on a radially inner side of the shifter.

81. The crimper for crimping the interventional instrument according to claim 80, wherein the shifter is block-shaped and is located between the inner wall of the housing and the ring gear.

82. The crimper for crimping the interventional instrument according to claim 81, wherein the driving portion faces a radially outer side of the shifter; and
an orientation of the locking portion is perpendicular to an orientation of the shifter.

83. The crimper for crimping the interventional instrument according to claim 81, wherein the shifter has a first groove and a second groove on two opposite sides in the axial direction of the ring gear, wherein the first groove accommodates part of the ring gear, the second groove accommodates the cam, and an inner wall of the second groove is the driving portion.

84. The crimper for crimping the interventional instrument according to claim 83, wherein the radially inner side of the shifter has a third groove, and a bottom wall of the third groove is the transmission portion; and
the shifter further comprises a post fixed in the third groove, and an end of the elastic member surrounds an outside of the post.

85. The crimper for crimping the interventional instrument according to claim 84, wherein the shifter has a top surface and an opposing bottom surface, and the locking portion is located on the top surface of the shifter and is located between the first groove and the third groove.

86. The crimper for crimping the interventional instrument according to claim 76, wherein the transmission member is a cam, the operation button and the cam are in a transmission fit through a plurality of transmission gears meshed with each other, and a rotation axis of the operation button, a rotation axis of the cam and rotation axes of the transmission gears are parallel to each other; and
depending on transmission sequence, an initial-stage transmission gear is coaxially arranged with the operation button, and a terminal-stage transmission gear is coaxially arranged with the cam.

87. The crimper for crimping the interventional instrument according to claim 73, wherein the shifter is located between two adjacent force-applying blocks in a circumferential direction of the instrument channel, and movement path of the shifter avoids the force-applying blocks.

88. The crimper for crimping the interventional instrument according to claim 86, wherein the crimper further comprises a locking member for locking the shifter at the corresponding working position, and the locking member directly or indirectly interferes with at least one of the following movable members:
the shifter;
the operation button;
the cam; and
the transmission gears.

89. The crimper for crimping the interventional instrument according to claim 88, wherein an inner wall of the housing has a plurality of locking slots for cooperating with the locking member, and the plurality of locking slots are distributed in a rotation or movement direction of a corresponding movable member in sequence.

90. The crimper for crimping the interventional instrument according to claim 89, wherein the locking member comprises:
a locking tongue for cooperating with a corresponding locking slot; and
a connecting arm connected between the locking tongue and the corresponding movable member and being deformable.

91. The crimper for crimping the interventional instrument according to claim 90, wherein the connecting arm extends in an arc shape, two ends of the connecting arm are connected to the corresponding movable member respectively, and the locking tongue is located at a top of the arc of the connecting arm.

92. A method for loading an interventional instrument, comprising:
providing a crimper which comprises:
a housing with a through instrument channel;
a plurality of force-applying blocks which are movably installed in the housing and distributed around the instrument channel, the plurality of force-applying blocks have relative gathered and separated states and are configured to contract and expand the instrument channel during switching the states; and
a ring gear which is rotatably matched with the housing and configured to synchronously drive the plurality of force-applying blocks to switch the states;
placing the interventional instrument in the instrument channel;
rotating the ring gear to drive the plurality of force-applying blocks to move synchronously to crimp the interventional instrument; and
transferring the crimped interventional instrument into a sheath for loading the interventional instrument.

93. The method for loading the interventional instrument according to claim 92, wherein the sheath is pre-positioned and aligned with the instrument channel.

94. The method for loading the interventional instrument according to claim 93, wherein depending on radial sizes of different sections of the interventional instrument, the interventional instrument is crimped by a stepwise crimping method.

95. The method for loading the interventional instrument according to claim 94, wherein the interventional instrument comprises a first section and a second section in its own axial direction, and a radial size of the first section is larger than that of the second section; and
the stepwise crimping method comprises:
inserting a mandrel into the interventional instrument and crimping the first section so that the radial size of the first section is approximately equal to the radial size of the second section; and
synchronously crimping the first section and the second section until the first section and the second section are in contact with the mandrel.

96. The method for loading the interventional instrument according to claim 95, further comprising inserting a first sheath of a delivery system into the interventional instrument, crimping the interventional instrument until it is in contact with the first sheath, and pushing the crimped interventional instrument into a second sheath of the delivery system.
